(19) Europäisches Patentamt
European Patent Office
Office européen des brevets

(11) EP 1 263 374 B1

(12) **EUROPEAN PATENT SPECIFICATION**

(45) Date of publication and mention of the grant of the patent:
**17.11.2004 Bulletin 2004/47**

(21) Application number: **01903417.2**

(22) Date of filing: **30.01.2001**

(51) Int Cl.[7]: **A61F 13/536**, A61F 13/537

(86) International application number:
**PCT/US2001/002971**

(87) International publication number:
**WO 2001/054641 (02.08.2001 Gazette 2001/31)**

(54) **ABSORBENT PRODUCTS WITH IMPROVED VERTICAL WICKING AND REWET CAPABILITY**

ABSORBIERENDER ARTIKEL MIT VERBESSERTEN VERTIKALEN DOCHT- UND RÜCKFEUCHTEEIGENSCHAFTEN

PRODUITS ABSORBANTS A CAPILLARITE VERTICALE ET A CAPACITES DE REHUMIDIFICATION AMELIOREES

(84) Designated Contracting States:
**AT BE CH CY DE DK ES FI FR GB GR IE IT LI LU MC NL PT SE TR**
Designated Extension States:
**AL LT LV MK RO SI**

(30) Priority: **31.01.2000 US 495530**
**12.06.2000 US 211091 P**

(43) Date of publication of application:
**11.12.2002 Bulletin 2002/50**

(73) Proprietor: **BKI Holding Corporation**
**Wilmington, DE 18901 (US)**

(72) Inventors:
- **GROSS, James, R.**
  **Cordova, TN 38018 (US)**
- **BOEHMER, Brian, E.**
  **Bartlett, TN 38135 (US)**
- **ERSPAMER, John, P.**
  **Lakeland, TN 38002 (US)**
- **BAKER, John, Perry**
  **Memphis, TN 38104 (US)**

(74) Representative: **Christiansen, Ejvind et al**
**Zacco Denmark A/S**
**Hans Bekkevolds Allé 7**
**2900 Hellerup (DK)**

(56) References cited:

| | |
|---|---|
| **WO-A-95/22952** | **US-A- 3 838 694** |
| **US-A- 3 993 820** | **US-A- 4 259 958** |
| **US-A- 5 262 223** | **US-A- 5 607 414** |
| **US-A- 5 843 055** | **US-A- 5 961 757** |

EP 1 263 374 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

FIELD OF THE INVENTION

The present invention relates to disposable absorbent hygiene products like diapers, sanitary napkins, surgical drapes, wound dressings, and to absorbent cores for use therein, and to a process for making these products with enhanced softness and fluid-handling capability. More particularly, this invention relates to a multi-layer airlaid absorbent structure having a discrete layer of compressible fibrous wicking material placed between a moisture impermeable backsheet and a fluid storage layer, and to a continuous airlaid process for the production thereof.

BACKGROUND OF THE INVENTION

Disposable absorbent hygiene products such as premium baby diapers, training pants, adult incontinence devices and feminine napkins are typically made with a cellulose fiber fluff-based absorbent core sandwiched between a liquid pervious top sheet and a low density acquisition or surge layer whose function is to allow the temporary storage and unobstructed passage of fluid to the absorbent core while acting as a barrier to the retransfer of liquid back to the skin of the user. and a liquid impervious backing sheet usually of plastic material, whose function is to contain the absorbed fluid and prevent it from passing through the absorbent core and soiling the undergarments of the wearer of the absorbent article. The acquisition layer typically comprises chemically stiffened cellulose fluff or bonded synthetic fibers, wherein the bonding is through the medium of thermoplastic binder fibers or powder or via the application of a latex binder.

The absorbent core of these absorbent articles is usually constructed of defiberized wood pulp with or without superabsorbent polymer granules. The absorbent core is typically formed on a pad-forming unit of a converting machine on a carrier tissue to facilitate processing. Some absorbent core forming units are equipped with layering capability in which a second discrete fluff layer may be laid over a primary fluff-based absorbent layer to form a multi-layer absorbent core. U.S. Patent No. 3,838,694 describes a multilayer absorbent pad, with a primary layer laid on a balt comprising loosely compacted fluff integrated with a densified fibrous layer. In these absorbent cores, the primary layer may include superabsorbent polymer granules. With regard to conventionally produced absorbent cores, reference is made to U. S. Patent Nos. 5,378,528, 5,128,082, 5,607,414, 5,147,343, 5,149,335, 5,522,810, 5,041,104, 5,176,668, 5,389,181, and 4,596,567. In regard to the superabsorbent polymer component of absorbent cores, it is known from U.S. Pat. Nos. 3,669,103 and 3,670,731 to crosslink carboxylic polyelectrolytes to create hydrogel-forming materials, now commonly referred to as superabsorbents, and to use such materials to enhance the absorbency of disposable absorbent articles.

Fluid distribution, which is defined as wicking in both the horizontal and vertical planes, is dependent on effective capillary diameter, among other factors. It is well known that the effective size of capillaries formed between adjacent fibers in a fibrous structure is determined by fiber size and the density or extent of compaction of the fibrous structure. In U.S. Patent Nos. 5,647,863, and 4,259,958 a combined storage and distribution member of an absorbent core is disclosed, that has higher capillary suction than a fluid acquisition member and a stain-indicating member. The storage/distribution layer not only draws the fluid insult out of the acquisition layer, but also preferably distributes the insult more in the longitudinal direction of the core than toward the sides. This preferential movement of the fluid is a result of alignment of the fibrous materials in the machine direction during wet processing. Wetlaid tri-component assemblies of chemically stiffened, curled, bulking fibers with high surface area fibers like eucalyptus and chemical binders or thermal bonding fibers, as disclosed in U.S. Patent Nos. 5,549,589, 5,800,416, and 5,843,055, are especially preferred for the distribution/storage layer of U.S, Patent No. 5,647,863. In U.S. Patent No. 5,009,650, a multi-layered cellulose fluff absorbent structure is disclosed in which the layers of cellulosic fiber differ in density or average pore size. In one aspect of the '650 disclosure, a separately-formed higher density or smaller pore size layer underlies at least a portion of a lower density layer to wick fluid from the fluid insult target area and transfer some of the fluid back to the lower density layer. Naturally, only those pores in the lower density layer which are immediately adjacent to the higher density layer and of a smaller pore size than the largest pores in the higher density layer will accept fluid from the higher density layer. Since the lower density layer is mainly devoid of fluid. rewet problems are lessened. Rewet occurs when fluid is expressed under pressure back through the diaper top sheet and contacts the skin of the wearer. While this wicking/storage layer can be achieved using the same fibers as in the lower density layer by simply increasing the density of the wicking layer. it can also be obtained by using smaller diameter fibers so the pore size between fibers is smaller even without increasing the density. Various hardwood fluff fibers, including eucalyptus, are suggested for the lower (wicking/storage) layer. In another aspect of U.S. Patent No. 5,009,650, a higher density layer containing superabsorbent granules is placed under the low-density fluff layer.

The wicking/storage layer comprises superabsorbent polymer granules mixed with fiber and sandwiched between high-density fluff layers. U.S. Patent No. 5,009,650 illustrates several product designs intended to achieve fluid distribution in absorbent products. However, in each case, fluid distribution and ultimate fluid storage occur in the higher density layer, which may contain superabsorbent polymer.

As a different way of controlling capillary size, a gradient of fiber sizes from large at the top to small at the bottom is disclosed in U.S. Patent No. 4,223,677 for fluid transfer into a disposable diaper. In an entirely different approach, U.S. Patent No. 5,188,624 teaches a liquid dispersion layer between an absorbent core and a backing sheet in which the liquid dispersion layer has a lower density than the insert. An enhanced vertical wicking rate is alleged for such a construction. In like manner, U.S. Patent No. 5, 401,267 interposes a lower density non-wicking layer between two high wicking layers and suggests that the inner layer transfers fluid from the first layer to the third layer. The second layer, however, retains fluid only when the third layer is saturated. U.S. Patent No. 4,573,988 teaches an ultrathin and lightweight (less than 136 gsm or 4 oz./yd$^2$), absorbent core in which a first, absorbent layer formed of synthetic staple fibers is held in a stabilized compressed state until released by wetting of the product. Fluid distribution in the x, y plane occurs mainly in a higher density wicking layer, which may be comprised of cellulose fibers. The first, absorbent layer contains superabsorbent polymer rendered tacky during the process so as to act as a temporary binder for the compressed synthetic fibers. When the superabsorbent begins to swell after a fluid insult, the first layer pops open so the swelling of the superabsorbent is not impeded and the capillary suction of the first layer becomes significantly lower than that of the second layer.

Effective wicking layers in absorbent products may be patterned or embossed to form discrete regions of higher density within an overall lower apparent density material. In U.S. Patent No. 3,938,522, a densified paper-like layer is formed on a cellulosic batt of wood pulp fibers by moistening the batt of fibers followed by heavy compaction. A densified layer created with moisture and pressure is commonly referred to as a "Burgeni" layer after the named inventor of U.S. Patent No. 3,017,304. The "Burgeni" layer disclosed in U.S. Patent No. 3, 938,522, is optionally formed with grooved compaction rolls to increase the available surface area of the paper-like layer and provide thickened ribs of densified material for transporting higher volumes of fluid than would be possible with a flat layer. The entire absorbent article is embossed on one or both sides to form spaced densified areas to enhance fluid spreading in U.S. Patent No. 4,443,512. Dry-laid or wet-laid fibrous webs of wood pulp fibers in U.S. Patent No. 4,612,231, are wetted with water and pressed between two heated cylinders, one of which is machined to impart a grid pattern into the fibrous web. A low density, soft, bulky, and absorbent paper sheet with a diamond pattern even after creping is described in U.S. Patent No.3. 905,863, in which the wet-laid sheet is pressed against a polymeric fabric of the desired texture. The densified areas in the pattern aid in fluid retention. Vertical wicking is not mentioned in U.S. Patent No.3,905.863. Similarly, in U.S. Patent No.3, 994,771, a wet-laid absorbent paper structure containing separate layers of long softwood fibers and short hardwood fibers is pressed against an open mesh drying/imprinting fabric to obtain softness and bulk. With these processes, as the density or degree of compaction of a material increases, so also does the bending modulus or stiffness of the material.

## SUMMARY OF THE INVENTION

A need exists in the industry to provide vertical wicking without loss of softness due to compaction of the materia). As the density or degree of compaction of a material increases, so also does the bending modulus or stiffness of the material. One consequence of the present invention is in divorcing overall product density and stiffness from the ability of the product to vertically wick fluid to high levels by including in a relatively low-density product a fibrous layer of a density conducive to effective wicking, preferably located near the moisture impervious outer cover. An essential feature of the wicking layers of this invention is the ability to effectively transfer the vertically wicked fluid to the adjacent fluid storage layer.

This invention is directed to an absorbent article made of a liquid permeable top sheet: a liquid impermeable back sheet; and an absorbent core disposed between the topsheet and the backsheet. The absorbent core is made on a continuous airlaid machine and includes an acquisition layer in effective fluid communication with the topsheet and disposed beneath the topsheet; a storage layer having absorbent capacity, the storage layer contacting the acquisition layer and being disposed beneath the acquisition layer; and a wicking layer having a density between 0.1 g/cc and 0.3 g/cc, the wicking layer contacting the storage layer, and being disposed beneath the storage layer; the wicking layer being made of compressible wood fibers. wherein the ratio of the vertical wicking height of the wicking layer to the vertical wicking height of the storage layer is greater than 1.25, but is preferably about 3.0 Additionally, this invention provides a method to make an absorbent article having a wicking layer with the ability to effectively transfer the vertically-wicked fluid to the adjacent fluid storage layer.

This invention provides an absorbent core comprising:

(1) an acquisition layer in effective fluid communication with
(2) a storage layer having absorbent capacity disposed beneath and contacting the acquisition layer, and
(3) a wicking layer disposed beneath and contacting the storage layer, comprising compressible hardwood pulp and having a density of from about 0.05 to about 0.4 g/cc, where the ratio of the vertical wicking height of the wicking layer to the vertical wicking height of the storage layer is equal to or greater than 1.25.

Within the scope of the aforementioned aspect of this invention is provided an absorbent article comprising:

(A) a liquid permeable top sheet,
(B) a liquid impermeable back sheet, and
(C) an absorbent core disposed between the topsheet and the backsheet. comprising:

(1) an acquisition layer disposed beneath the topsheet and in effective fluid communication with the topsheet and in effective fluid communication with
(2) a storage layer having absorbent capacity disposed beneath and contacting the acquisition layer, and
(3) a wicking layer disposed beneath and contacting the storage layer. comprising compressible hardwood pulp and having a density of from 0.05 to 0.4 g/cc.

where the ratio of the vertical wicking height of the wicking layer to the vertical wicking height of the storage layer is equal to or greater than 1.25.

This invention provides a method of making an absorbent core comprising:

(A) forming a wicking layer comprising compressible hardwood fibers,
(B) compressing the wicking layer to a density of between 0.05 to 0.4 g/cc, and, optionally, imprinting a compression pattern on the wicking layer,
(C) forming a storage layer having absorbent capacity in effective fluid communication with the wicking layer, and
(D) forming an acquisition layer in effective fluid communication with the storage layer,

where the ratio of the vertical wicking height of the wicking layer to vertical wicking height of the storage layer is equal to or greater than 1.25.

The present invention also provides a method of making an absorbent article comprising:

(A) providing a liquid impervious back sheet,
(B) forming a wicking layer comprising compressible hardwood fibers,
(C) compressing the wicking layer to a density of between 0.05 to 0.4 g/cc, and, optionally, imprinting a compression pattern on the wicking layer.
(D) forming a storage layer having absorbent capacity in effective fluid communication with the wicking layer,
(E) forming an acquisition layer in effective fluid communication with the storage layer, and
(F) providing a liquid pervious top sheet in effective fluid communication with the acquisition layer,

where the ratio of the vertical wicking height of the wicking layer to vertical wicking height of the storage layer is equal to or greater than 1.25.
Within the scope of this aspect of the invention is the core made by the aforementioned method.

This invention also provides a method of making an absorbent article comprising:

(A) providing a liquid impervious back sheet,
(B) forming a wicking layer comprising compressible hardwood fibers,
(C) compressing the wicking layer to a density of between 0.05 to 0.4 g/cc, and, optionally, imprinting a compression pattern on the wicking layer,
(D) forming a storage layer having absorbent capacity in effective fluid communication with the wicking layer,
(E) forming an acquisition layer in effective fluid communication with the storage layer, and
(F) providing a liquid pervious top sheet in effective fluid communication with the acquisition layer,

where the ratio of the vertical wicking height of the wicking layer to vertical wicking height of the storage layer is equal to or greater than 1.25. Within the scope of this aspect of the invention is the absorbent article made by the aforementioned method.

In another embodiment, this invention provides an absorbent core comprising:

(1) an acquisition layer in effective fluid communication with
(2) a storage layer having absorbent capacity disposed beneath and contacting the acquisition layer, and
(3) a wicking layer disposed beneath and contacting the storage layer, comprising compressible hardwood pulp.

The core is made by a method of making an absorbent core comprising:

(A) forming a wicking layer comprising compressible hardwood fibers.
(B) compressing the wicking layer to a density of between 0.05 to 0.4 g/cc, and, optionally, imprinting a compression pattern on the wicking layer.
(C) forming a storage layer having absorbent capacity in effective fluid communication with the wicking layer, and
(D) forming an acquisition layer in effective fluid communication with the storage layer.

In yet another embodiment, this invention provides an absorbent core comprising:

(1) an acquisition layer in effective fluid communication with
(2) a storage layer having absorbent capacity disposed beneath and contacting the acquisition layer, and
(3) a web imprinted wicking layer disposed beneath and contacting the storage layer, comprising compressible wood pulp in which there is a pattern of densified regions and less densified regions in the fibrous web. The core is made by a method of making an absorbent core comprising:

(A) forming a wicking layer comprising compressible wood fibers,
(B) compressing the wicking layer to a density of between 0.05 to 0.4 g/cc, where densification of the wicking layer is done between a forming or transfer fabric and a compaction roll or between a patterned compaction roll and a smooth roll or between two patterned compaction rolls to form a web imprinted wicking layer with a pattern of densified regions and less densified regions in the fibrous web,
(C) forming a storage layer having absorbent capacity in effective fluid communication with the wicking layer, and
(D) forming an acquisition layer in effective fluid communication with the storage layer.

## BRIEF DESCRIPTION OF THE FIGURES

Fig. 1 is a cross-sectional view of the absorbent structure of an embodiment of the present invention.
Fig. 2 is a top view of a web imprinted wicking member of an embodiment of the present invention.
Fig. 3 is a side view of a web imprinted side member of an embodiment of the present invention.
Fig. 4 is a graph of wicking height versus density.
Fig. 5 is a graph of rewet results.
Fig. 6 is a graph of rewet results.
Fig. 7 is a graph of rewet results.
Fig. 8 is a graph of stain size results.
Fig. 9 (a) and (b) depicts perspective views of partially densified embodiments of the present invention.

## DETAILED DESCRIPTION OF THE PREFERRED EMBODIMENTS

It has now been discovered that by incorporating an independent wicking layer into a disposable absorbent article, wicking height, fluid retention and rewet can be improved without compromising product softness. This wicking layer efficiently transports fluid vertically for a greater distance than is possible in a unitary or homogeneous structure of comparable overall density and also distributes the fluid laterally into an adjacent fluid storage layer and does so at an overall density and product stiffness such that the product remains soft, flexible, and comfortable to wear. The wicking layer is plivslcally independent of the other layers of the absorbent core, but is still in fluid communication with the adjacent storage layer. It is hypothesized that the osmotic pressure (fluid suction power) of the superabsorbent material in the storage layer is the driving force behind the effective transfer of the vertically-wicked fluid from the higher density or smaller pore size wicking layer back into the lower density storage layer. Superabsorbent polymers, being typically the sodium salt of poly (acrylic acid), are highly ionic and consequently exhibit high osmotic pressure. This high osmotic driving force overcomes the natural tendency for fluid to remain in regions of smaller pore size capillaries.
In one embodiment of this invention, this improvement can be achieved by constructing the wicking layer from compressible wood pulp fibers such as hardwood pulp, typified by eucalyptus, or from softwood pulp treated to be readily compressible. It should be noted that mixtures of fibers, including hardwood and chemically treated softwood fibers, as well as minor amounts of synthetic fibers, may be used. The wicking layer typically contains from about 50 percent by weight to about 99.9 percent or 100 percent by weight of compressible wood pulp fibers, more desirably from about 80 percent to about 100 percent by weight, and preferably from about 90 percent to about 100 percent by weight of compressible wood pulp fibers, and, optionally, from about 0.1 percent to about 50 percent by weight of synthetic fibers, more desirably from about 0.1 percent to about 20 percent by weight, and preferably from about 0.1 percent to about 10 percent by weight, of synthetic fibers. The wicking layer may also contain binders, such as, for example, latex binders, or binding may be accomplished thermally with minor amounts of one or more synthetic fibers, such as, for example, a bicomponent fiber, or with a powder, such as, for example, polyethylene powder.

In an alternative embodiment, various synthetic fibers can be substituted for the wood fibers of the wicking layer. Desirably, these are wettable fibers having a diameter of from about 5 microns to about 15 microns and a length of from about 0.5 mm to about 2.5 mm. Fibers of rayon, acrylic, polyester, polyamide and polyolefin, including polyethylene and polypropylene, may be suitable. A surfactant treatment may improve the wettability of the fibers.

In a further embodiment of this invention, the wicking layer is densified to the desired degree for superior vertical wicking prior to forming the remainder of the product layers upon it, whereby the wicking heights achievable by the entire product are thereby enhanced. The measured density of the wicking layer will depend upon the pressure applied during the measurement. After densification, the density of the wicking layer under an applied pressure of 4 $g/cm^2$ desirably is in the range from about 0.05 g/cc to about 0.4 g/cc, more desirably from 0.06 to 0.3 g/cc; even more desirably from 0.1 to 0.3 g/cc or between 0.1 to 0.3 g/cc. More particularly, with a hardwood pulp fiber such as eucalyptus, the density of the wicking layer is preferably from about 0.05 to about 0.2 g/cc and more preferably about 0.15. With compressible softwood fibers the density desirably is from about 0.20 to about 0.40, and more preferably is about 0.25. At these density ranges the wicking layer has limited absorbent capacity. Further, in an alternative embodiment, the density of the wicking layer under an applied pressure of 11 $g/cm^2$ desirably is in the range from about 0.08 g/cc to about 0.4 g/cc, more desirably from 0.08 to 0.2 g/cc.

In yet another embodiment of the invention, the densification of the wicking layer is done between the forming or transfer fabric and a compaction roll or between a patterned compaction roll and a smooth roll or between two patterned compaction rolls to impose a pattern of densified regions and less densified regions in the fibrous web. The various compaction rolls may be heated. In this embodiment, it has been surprisingly found that untreated softwood fibers can be used. FIGS. 2 and 3 show views of a web imprinted wicking layer. The portion of the wicking layer that has been compacted is shown as a plurality of stripes 22 and the noncompacted portion of the wicking layer is shown as 24. The density of the web compacted stripes ranges from about 0.1 g/cc to 0.5 g/cc.

Although most of these procedures would be effective on wet-laid cellulose pulp sheets, a continuous airlaid web-forming process is ideally suited to the execution of all the embodiments of this invention. In wet-laid sheet forming, a highly dilute slurry of fibers in water is deposited on a rapidly moving screen. In airlaid sheet forming, the individualized fibers are dispersed in a stream of air and deposited dry on a moving screen. Various aspects of the airlaid process are disclosed in U.S. Patent Nos. 5,068,079; 5,269,049; 5,693,162; 5,922,163; 6,007,653; 5,927,051; 5,956,926; 5,966,905; 5,921,064; 5,987,851; 6,009,689; 6,067,775; 5,885,516; 5,028,224; 5,227,107; 5,316,601; 4,908.175; 4,927,582; 5.429.788; 5,445,777; 5,558,832.

In a preferred embodiment of this invention, the absorbent core is a unitary absorbent core produced in a series of unit operations in a continuous process, preferably an airlaid process.

Wicking can be improved along the machine-direction of the fibrous weh by embossing high-density lanes into the web. This method imparts cross-machine density gradients to the web. This method does not negatively affect web stiffness as much as the method ofdensifying the entire web. However, this method also significantly reduces the void volume of the web, in that this method calls for embossing through the entire web.

The present invention calls for using an airlaid process with multiple heads and between-head embossing to produce a fibrous web with improved wicking. Wicking can be improved with the present invention without sacrificing large amounts of web void volume. Thus with the present invention wicking and fluid distribution can be improved without a large negative effect on fluid acquisition rate.

The web according to the present invention can be manufactured, for example, using an airlaid line with two forming heads. The first forming head can be used to uniformly form a lower stratum of a two-strata unitary absorbent core. Next, an embossing roll can be used to emboss the lower stratum against the forming wire of the airlaid line, thus placing high-density wicking lanes into the lower stratum. Next, the second forming head can be used to uniformly form the upper stratum of a two-strata unitary absorbent core. Finally, the entire web can be densified in a calendar stack to a uniform thickness. Densifying the two-strata unitary absorbent core to a final, uniform thickness ensures that channels of relatively low density are present in the upper stratum of the core, directly above the high-density wicking lanes of the lower stratum. Thus, high-density wicking lanes can be placed in unitary absorbent cores, while at the same time providing for relatively low-density channels to preserve void volume. See Figures 9(a) and (b).

Additionally, the wood pulp fibers can be treated by imprinting the wicking layer with the pattern of the forming or transfer fabric using a compaction roll, which may be heated, or embossing the wicking layer with a patterned and, optionally, heated compaction roll. This treatment is referred to as web treatment. Heated compaction rolls are typically smooth-faced steel drums equipped with an internal heat source and a hydraulic means of applying pressure to the compaction roll against another steel or hard rubber roll. When the compaction roll is engraved with a pattern which, under pressure. transfers to the web being compacted, the engraved roll is typically known as an embossing roll and may be heated. Thus, the selection of pulp type and/or web treatment allows the material of this invention to physically transport fluid vertically to an extent not practicable in homogeneous (nonlayered) structures due to stiffness limitations for product comfort. An additional feature of this invention is that the softness and hence comfort of the final absorbent products is not compromised by the densities needed for superior vertical wicking.

Now referring to FIG. 1, a cross-sectional view of an airlaid absorbent article is shown with moisture permeable topsheet 10, acquisition layer 11, storage layer 12. wicking layer 13 and moisture impermeable backsheet 14. The topsheet 10 is liquid pervious and should be flexible and nonirritating to the skin. The acquisition layer 11, storage layer 12 and wicking layer 13 form the absorbent core 15. The absorbent core 15 is used to collect bodily fluids such as menses or urine.

The topsheet 10, if employed, presents a body-facing surface which is compliant, soft-feeling. and non-irritating to the wearer's skin. Further, the topsheet 10 is sufficiently porous to he liquid permeable, permitting liquid to readily penetrate through its thickness. A suitable topsheet 10 may be manufactured from a wide range of materials, such as porous foams, reticulated foams, apertured plastic films, natural fibers, such as, for example, wood or cotton fibers, synthetic fibers, such as, for example, polyester or polypropylene fibers, or a combination of natural and synthetic fibers. The topsheet 10 is typically employed to help isolate the wearer's skin from liquids held in the absorbent structure.

The topsheet 10 can have a basis weight, ranging from about 10.0 gsm to about 100 gsm. and a density of between about 0.05 g/cc and about 0.5 g/cc. The topsheet 10 can be treated with a selected amount of surfactant, or otherwise processed to impart the desired level of wettability and hydrophilicity, If a surfactant is used, it can be an internal additive or applied to the layer by any conventional means, such as spraying. brush coating and the like.

Various woven and nonwoven fabrics can be used for the topsheet 10. For example, the topsheet may be composed of meltblown or spunbonded web of polyolefin fibers. The topsheet may also be a bonded-carded-web composed of natural and/or synthetic fibers.

The absorbent core may be manufactured in a variety of shapes. The core 15 is preferably conformable and nonirritating to the skin. The acquisition layer 11 is positioned between the topsheet 10 and the storage layer 12. The acquisition layer 1I functions to quickly collect and temporarily hold bodily fluids that have been deposited thereon or which have been traversed through the topsheet 10. Additionally, the acquisition layer 11 functions to transport those bodily fluids to the underlying storage layer 12. The acquisition 11 and storage 12 layers are thus in effective fluid communication. The acquisition layer 11 must have sufficient capillary suction to draw bodily fluids through the topsheet 10 but not have excessive fluid retention to make it difficult for the storage layer 12 positioned below the acquisition layer 11 to deabsorb the acquisition layer 11. Suitable material for the acquisition layer may include cross-linked cellulose fibers, synthetic fibers, or combinations thereof. The density of the acquisition layer is between 0.04 to 0.1 g/cc.

The storage layer 12 functions to receive and ultimately contain bodily fluids passing through the acquisition layer 11. The acquisition 11 and storage 12 layers are thus in effective fluid communication. The density of the storage layer is between 0.05 to 0.25 g/cc. Examples of suitable materials for the storage layer 12 include synthetic or chemically treated cellulosic fibers and wood pulp and superabsorbent materials. The wicking layer 13 is positioned below the storage layer 12 and above the moisture impermeable backsheet 14. The wicking layer 13 functions to draw fluid out of the storage layer, to wick it to another less saturated area, and then to transfer a substantial portion of the fluid back to the storage layer 12. The storage layer 12 and the wicking layer 13 are thus in effective fluid communication.

Various acquisition layers and storage layers have been disclosed which are suitable for use in this invention, such as, for example, the acquisition layer and the storage layer of U.S. Ser. No. 09/325,764, and corresponding PCT/US00/16001. Various materials use in the construction of the acquisition layer and the storage layer and the resultant properties and characteristics of the layers are disclosed in U.S. Patent Nos. 5,147,343; 5,378,528; 5,795,439; 5,807,916; 5,849,211; 2,929,154; 3,224,986; 3,332,909; and 4,076,673.

The backsheet 14 is substantially impermeable to liquids and is typically manufactured from a thin plastic film, or other flexible liquid-impermeable material. As used in the present specification, the term "flexible" refers to materials which are compliant and which will readily conform to the general shape and contours of the wearer's body. The backsheet 14 prevents the exudates contained in the absorbent structure from wetting articles such as bed sheets and over garments that contact the finished product. The backsheet 14 may be, for example, a polyethylene film having a thickness of from about 0.012 mm (0.5 mil) to 0.051 mm (2.0 mils). Alternatively, the backsheet may be a woven or nonwoven fibrous web layer that has been constructed or treated to impart the desired level of liquid impermeability. Other alternative constructions for the backsheet 14 include laminates formed of a woven or nonwoven fabric and thermoplastic film.

The backsheet 14 may optionally be composed of a "breathable" material which permits vapors to escape from the absorbent structure while still preventing liquid exudates from passing through the backsheet. The backsheet can also be embossed and/or matte finished to provide a more aesthetically pleasing appearance.

In a preferred embodiment of this invention, the wicking layer comprises hardwood fiber, such as, for example, eucalyptus, birch, aspen, maple, cotton wood, willow, oak, beech, poplar and basswood, preferably, one or more of the several species of eucalyptus. In another embodiment of this invention, the wicking layer comprises a fluff pulp, either a hardwood pulp or a chemically treated softwood pulp , which is defined as a softwood pulp which has been softened or plasticized to be inherently more compressible than unmodified fluff pulp fibers. The same pressure applied to a plasticized fluff pulp web will result in higher density than when applied to an unmodified fluff pulp web. Additionally, the densified web of plasticized cellulose fibers is inherently softer than a similar density web of unmodified fiber of the same wood type. Softwood pulps may be made more compressible using cationic surfactants as debonders to disrupt interfiber associations. Examples of debonders are disclosed in U.S. Patent Nos. 4,432,833, 4,425,186 and 5,776,308.

Plasticizers for cellulose, which can be added to a pulp slurry prior to forming wetlaid sheets, can also be used to soften pulp, although they act by a different mechanism than debonding agents. Plasticizing agents act within the fiber, at the cellulose molecule, to make flexible or soften amorphous regions. The resulting fibers are characterized as limp. Since the plasticized fibers lack stiffness, the comminuted pulp is easier to densify compared to fibers not treated with plasticizers.

Plasticizers include polyhydric alcohols such as glycerol; low molecular weight polyglycol such as polyethylene glycols and polyhydroxy compounds. These and other plasticizers are described and exemplified in U.S. Patent Nos. 4,098,996, 5547,541 and 4731,269. Ammonia, urea, and alkylamines are also known to plasticize wood products, which mainly contain cellulose (A. J. Stamm, Forest Products Journal 5(6):413, 1955.

Desirably, the ratio of the wicking height of the wicking layer to the wicking height of the storage layer (homogeneous construction) is equal to or greater than 1.25, more desirably, equal to or greater than 1.50, preferably, equal to or greater than 1.75, and more preferably, equal to or greater than 2.0, and even more preferably, equal to or greater than 3.0.

An unanticipated advantage of using a hardwood pulp wicking layer under an absorbent structure is in the improvement of rewet performance. Rewet or flowback is the interstitial fluid held in an absorbent structure, which may be released back through the topsheet under pressure. Lower rewet relates to better dryness for the user of the product. That a thin layer of fibers under a much heavier fluid storage layer could impact the amount of fluid expressed back through the acquisition layer and topsheet was surprising. It is theorized that since the rewet test is performed under less than saturated conditions, the stronger capillary suction power of the low basis weight wicking layer partially drains the larger capillaries in the partially saturated adjacent fluid storage layer thereby reducing the volume of fluid available for expression from the structure under the modest pressure of the rewet test.

The absorbent cores of this invention desirably have a rewet of about 2.5 g or less. more desirably of about 2.0 or less, preferably of about 1.5 g or less. more preferably of about 1.0 g or less. and still more preferably, the absorbent core has a rewet of about 0.5 g or less.

<u>Examples</u>

<u>Test Methods for Wicking Examples</u>

Except as may be noted in a specific test method, all tests should be conducted at 23° C (73° F) and 50% relative humidity and all samples should be conditioned at this temperature and humidity for at least two hours prior to testing.

A. Density

Density (grams per cubic centimeter - g/cc) may be calculated from the basis weight (grams per square meter - gsm) and the caliper (centimeters - cm) as measured under a given confining pressure using the formula:

$$\text{Density (g/cc)} = \text{Basis Weight (g/m}^2\text{)}/[10{,}000\ \text{cm}^2/\text{m}^2 \times \text{Caliper(cm)}]$$

Basis Weight (BW) is expressed in grams per square meter, density in grams per cubic centimeter.

B. Caliper

Caliper or thickness is measured as follows: At least three measurements are taken on different parts of a sample using a digital or analog thickness gauge made by AMES of Waltham, Mass. The measurements are averaged. The gauge has a 4 cm diameter foot and is equipped with a 50-gram weight so the pressure applied to the sample is 4 grams/$cm^2$. Thickness is measured in inches and converted to centimeters as needed for calculations.

C. Vertical Wicking

Vertical wicking is measured by hanging the absorbent article or core vertically and dipping into a shallow pan of 0.9% saline. The pan is equipped with a constant leveling device to maintain the level of saline. The vertical rise of the saline above the liquid level in the pan is measured after 30 minutes. Samples are measured in duplicate and the results averaged. The units of vertical wicking are in millimeters.

The invention is illustrated by a series of experiments in which diapers and other like articles were made and tested. The experimental variables were the density of the overall product, the density of the wicking layer. the type of cellulose fiber used in the wicking layer, and the creation of a densified pattern in the wicking layer. Other elements in the overall absorbent structure. such as the acquisition layer and the storage layer, were held constant for the purpose of illustrating the advantages of the particular wicking layers of this invention.

The wicking layers, absorbent cores, and acquisition layers were prepared sequentially on a conventional laboratory air forming handsheet apparatus producing a pad of 35.6 cm (14 inches) square, sufficient, after trimming, to yield four test samples of 7.6 cm by 22.9 cm (3 by 9 inches) or three samples of 7.6 cm by 25.4 cm (3 by 10 inches). This laboratory preparation simulated the type of product that can be produced continuously in a single pass on a conventional airlaid machine with at least three forming heads. It is understood that the several layers constituting an entire absorbent product could be separately formed and assembled by a lamination process to achieve a comparable product. However, such a product lacks certain advantages obtained in preparing a multilayer product in a continuous airlaid process. In the first step, simulating the first forming head of an airlaid line, the wicking layer is deposited on a carrier tissue or directly on the forming wire. If the wicking layer is densified at this time, the use of a between-the-heads compaction roll is simulated. Otherwise, the fluid storage layer is laid down by the second forming head followed by the acquisition layer by the third forming head. The entire composite is sprayed with a latex binder, dried and pressed to the final density for the wicking measurement. The final density being between 0.1 to 0.3 g/cc.

In the following experiments, an acquisition layer comprises 84 gsm of crimped polyester staple fiber (Type T-224 from Hoechst-Trevira of Salisbury. NC) to which is applied a latex binder (Airflex AF-181 from Air Products and Chemicals of Allentown PA). The absorbent cores, over which the acquisition layers are formed, all contained 161 gsm of Southern softwood kraft cellulose fluff (Foley fluff from Buckeye Technologies Inc. of Memphis. TN) and 375 gsm of commercial acrylic acid-based superabsorbent (FAVOR SXM70) available from Stockhausen Inc. of Greensboro. NC. The wicking layers used in the following working examples were prepared with a target basis weight of at least 150 gsm. The compressible pulp ND416 is available from Weyerhaeuser Corporation of Tacoma, WA. Sheeted bleached eucalyptus fiber is available from Sappi Saiccor of Johannesburg, South Africa and Aracruz Celulose (USA) Inc. of Raleigh, NC.

In the homogeneous construction, the storage layer was the same as in the other products and there is an acquisition layer but no separate wicking layer. When the pre-densification of the wicking layer between forming heads one and two was simulated in the laboratory (Examples 1-3), the wicking layer was formed and pre-densified to 0.30 g/cc and then the remainder of the structure was built upon it and compressed to the final overall density. The following samples were prepared and the vertical wicking results are shown in Table 1.

Examples 1-3 in Table 1 show the vertical wicking performance of the wicking layer of this invention and the same combined with a fluid storage and fluid acquisition layers to represent an absorbent product. Performance of a product without the wicking layer of this invention is given by the homogeneous construction, which included only the acquisition layer and the fluid storage layer. The sequential formation is a simulation of the kind of product produced on an airlaid line with three forming heads with web compaction after the heads. The pre-densified wicking layer construction simulates an airlaid line with three forming heads and a compaction roll between heads one and two to bring the wicking layer to a density of 0.3 g/cc prior to forming the other layers on it. The density values in Table I are for the overall constructions, whether single layer. bilayer, or trilayer.

Table I -

| Examples 1-3 Vertical Wicking | | | | | | | | | |
|---|---|---|---|---|---|---|---|---|---|
| | | Wicking Layer Alone | | Homogeneous No Wicking Layer | | Sequential Formation | | Pre-densified Wicking Layer | |
| Example | Fiber Type | Density g/cc | Wicking mm | Density g/cc | Wicking mm | Density g/cc | Wicking mm | Density g/cc | Wicking mm |
| 1 | Foley | 0.272 | 126 | 0.204 | 99 | 0.244 | 127 | 0.247 | 142 |
| 2 | ND416 | 0.268 | 152 | 0.192 | 102 | 0.251 | 137 | 0.251 | 150 |

Table I - (continued)

| Examples 1-3 Vertical Wicking | | | | | | | | | |
|---|---|---|---|---|---|---|---|---|---|
| | | Wicking Layer Alone | | Homogeneous No Wicking Layer | | Sequential Formation | | Pre-densified Wicking Layer | |
| Example | Fiber Type | Density g/cc | Wicking mm | Density g/cc | Wicking mm | Density g/cc | Wicking mm | Density g/cc | Wicking mm |
| 3 | Eucalyp | 0.259 | 199 | 0.200 | 121 | 0.249 | 158 | 0.247 | 165 |

The examples in Table 1 show that at essentially the same densities for a given layer or construction, vertical wicking height increases based on fiber type, in the order: Foley fluff < ND416 < eucalyptus. In Examples 1-3, the ratios of the wicking height of the wicking layer to that of the storage layer (homogeneous construction) are 1.27, 1.49, and 1.64, respectively.

The simulated pre-densification method to fabricate the article produces higher vertical wicking heights than the simple sequential formation approach in which the density of the wicking layer is not controlled but is determined by the compression applied to establish the overall density of the structures. The lowest wicking performance was displayed by the homogeneous construction, lacking a discrete wicking layer.

Table 2 (Examples 4-15) further illustrates the effect of the density of a wicking layer on the vertical wicking measurement. A compressible fluff was made in situ, Examples 13-15, by spraying Foley pulp sheet with 4% by weight glycerin just before fiberization. The laboratory airlaid handsheet apparatus was again used for sample preparation followed by compaction in a laboratory press.

Table 2 -

| **Wicking Layers and Density** | | | | | |
|---|---|---|---|---|---|
| Example | Fiber Type | Thickness mm | BW gsm | Density g/cc | Wicking Height mm |
| | | | | | |
| 4 | Eucalyptus | 0.064 | 161 | 0.250 | 199 |
| 5 | Eucalyptus | 0.056 | 177 | 0.318 8 | 209 |
| 6 | Eucalyptus | 0.048 | 173 | 0.358 | 214 |
| 7 | ND 416 | 0.086 | 169 | 0.196 | 108 |
| 8 | ND 416 | 0.058 | 166 | 0.268 | 152 |
| 9 | ND 416 | 0.046 | 171 | 0.375 | 217 |
| 10 | Foley Fluff | 0.064 | 175 | 0.272 | 126 |
| 11 | Foley Fluff | 0.051 | 169 | 0.339 | 200 |
| 12 | Foley Fluff | 0.046 | 168 | 0.369 | 233 |
| 13 | Pulp/Glycerin | 0.061 | 167 | 0.274 | 185 |
| 14 | Pulp/Glycerin | 0.053 | 169 | 0.323 | 208 |
| 15 | Pulp/Glycerin | 0.046 | 167 | 0.367 | 218 |

Table 2 shows that even the unmodified Foley fluff can achieve high vertical wicking values at high density. Unfortunately, the stiffness of the wicking layer at greater than 0.3 g/cc density is such that it may not be acceptable in a disposable product intended to conform to the human body. The stiffness ranking of these materials is Foley fluff > ND416 ~ Pulp/glycerin > eucalyptus.

The impact of patterning the densified wicking layer is demonstrated in Table 3 using the compressible pulp ND416 available from Weyerhaeuser. On an airlaid pilot line, a 150-gsm web was formed on an 18-gsm-carrier tissue and subjected to compaction to an initial density of 0.3 g/cc using a smooth compaction roll heated to 90°C. The weave of the transfer fabric was impressed into the cellulose web. This web was used as the wicking layer in a laboratory-made 800-gsm-diaper product containing 375 gsm of superabsorbent (Example 16). In Example 17 the same imprinted wicking layer was cut into 0.25-inch strips and placed 0.25 inch apart below the storage layer in a like diaper construction. In Example 18, the same imprinted wicking layer was cut into 0.5-inch strips and placed 0.5 inch apart below the storage layer. In Example 19, the 150 gsm wicking layer was made in the laboratory and pressed against a piece of the transfer fabric from the pilot plant to an apparent density of 0.3 in a laboratory press equipped with a grooved plate having 0.375 inch wide grooves 0.25 inch deep and 0.25 inch apart (See Figs. 2 and 3). The sample and plate were pre-heated in an oven to 150°C. This wicking layer was included in an 800-gsm-diaper product as before.

Table 3 -

| Patterned Wicking layers | | | | |
|---|---|---|---|---|
| Example | Density g/cc | Vertical Wicking, mm In densified lane | Vertical Wicking, mm In Storage Layer | Fluid Retention, g |
| 16 | 0.15 | Not applicable | 254 | 149 |
| 17 | 0.252 | 206 | 130 | 129 |
| 18 | 0.249 | 208 | 95 | 125 |
| 19 | 0.25 | 224 | 148 | 179 |

Examples 16-19 show that the interface area between layers is important for net fluid acquisition. The planar wicking layer in Example 16 wicked the entire length of the 10-inch sample. Examples 17 and 18 with the 6.4 mm (0.25-inch) and 13 mm (0.5 inch) wicking strips have half the contact area between the wicking layer and the storage layer as in Example 16. The strips alone raised the saline level over 200 mm, but failed to adequately transfer this fluid to the storage layer as evidenced by the low vertical height in the storage layer and low grams of saline retained. Example 19 with regions of intermediate density between the densified lanes efficiently moved fluid into the storage layer to a respectable height of 148 mm. In examples 17-19, the ratios of the wicking height of the wicking layer to that of the storage layer are 1.58, 2.19, and 1.51, respectively.

In Example 20, the effect of imprinting a pattern in the wicking layer is quite evident when a smooth wicking layer prepared in the laboratory is pressed to different densities for vertical wicking measurement. Extrapolating the straight line connecting the three points in FIG. 4 shows that a density of about 0.5 g/cc is needed to reach 300 mm wicking height in 30 minutes. The pilot line produced wicking layer used in the diaper construction of Example 16 wicked to 300 mm when tested alone at a density of 0.25 g/cc. This vertical wicking height suggests that in the densified regions of the fabric pattern, the density apparently equals or exceeds 0.5 g/cc. The imprinted web was surprisingly soft and conformable.

While it is important for product development for samples made at a certain density to be tested immediately, in the practical sense, materials must deliver the desired properties after prolonged storage and particularly after an absorbent article has been worn on the body for some time. During the use of absorbent products. motion of the wearer may tend to disrupt the carefully engineered structure so that the requisite densities are no longer present. In Example 21, a 5.1 cm by 46 cm (2 inch by 18 inch) strip of the fabric-imprinted 150 gsm wicking layer of Example 16 is subjected to repeated crushing by hand and then checked for apparent density and wicking performance along side a strip of the same web which had not been so handled. The results of wicking height versus time are shown in Table 4.

Table 4 -

| Vertical Wicking Rate | | | | | | |
|---|---|---|---|---|---|---|
| Sample | Density, g/cc | 5 min | 10 min. | 15 min. | 20 min. | 30 min. |
| "As Is" | 0.25 | 180 mm | 228 mm | 255 mm | 270 mm | 300 mm |
| Softened | 0.12 | 150 mm | 170 mm | 180 mm | 200 mm | 210 mm |

Table 4 clearly shows that even the "softened" imprinted compressible pulp wicking layer managed to raise 0.9% NaCl to a height of 210 mm in 30 minutes. It was able to do this despite an apparent density of only 0.12 g/cc and extreme softness from the mechanical working.

These are representative of articles formed in accordance with the invention; however, it will be clear to those skilled in the art that the present invention may be incorporated in other devices for the absorption of aqueous materials.

Test methods for Rewet Examples

Except as may be noted in a specific test method, all tests should be conducted at 23° C (73° F) and 50% relative humidity. All samples should be conditioned at this temperature and humidity for at least two hours before testing.

A. Basis weight

Basis weight is the weight of the sample per unit area and is expressed in grams per square meter ($g/m^2$ or gsm).

B. Thickness

Thickness is measured using an analog thickness gauge (B.C. Ames Co., Waltham, MA). The gauge has a 4.1-cm diameter foot and is equipped with a 150-gram weight so that the pressure applied to the sample is 11.4 $g/cm^2$. Thickness is measured in inches and is converted to centimeters as needed for calculations.

C. Density

Density can be calculated from the basis weight and the thickness under a given confining pressure using the formula:

$$\text{Density (g/cc)} = \text{Basis weight (gsm)} / [10{,}000\ cm^2 / m^2 \times \text{Thickness (cm)}]$$

Density is expressed in grams per cubic centimeter (g/cc).

D. Synthetic menstrual fluid

The synthetic menstrual fluid used in this work contains the following ingredients in the designated amounts:

- Deionized water 903.3 g
- Sodium chloride 9.0 g
- Polyvinylpyrrolidone 122.0 g
- Biebrich Scarlet 4.0 g

Total solution volume 1 liter

Biebrich Scarlet (red dye) can be obtained from Sigma Chemical Co.. St. Louis, MO. Polyvinylpyrrolidone (PVP, weight-average molecular weight approximately 55,000) can be obtained from Aldrich, Milwaukee, WI. Sodium chloride (ACS grade) can be obtained from J.T. Baker, Phillipsburg, NJ. The dry ingredients are mixed in water for at least two hours to ensure complete dissolution. The solution temperature is adjusted to 22°C exactly. Sixteen milliliters of solution is pipetted into the UL adapter chamber of a Brookfield Model DV-II+ viscometer (Brookfield Engineering Laboratories, Inc., Stoughton, MA). The UL spindle is placed into the chamber and the viscometer speed is set to 30 rpm. The target viscosity is between 9 and 10 centipoise. Viscosity can be adjusted with additional water or PVP.

E. Combination acquisition, rewet, stain size test

Equipment:

- Electronic balance (± 0.01 g precision)
- Fluid intake tester (FIT, Buckeye "BU144-97" design)
- Grade S22 blotter paper, 10.16 cm x 24.13 cm (4 in. x 9.5 in.)
- Weight. 8408.5 g, 10.16 cm x 24.13 cm (4 in. x 9.5 in.)
- Latex foam. 10.16 cm x 24.13 cm x 3.81 cm (4 in. x 9.5 in. x 1.5 in.)
- Ruler, scaled in millimeters
- Synthetic menstrual fluid

Topsheet, spunbond polypropylene, 22 gsm, 25.4 cm x 10.16 cm (10 in. x 4 in.)

Latex foam can be obtained from Scott Fabrics, Memphis, TN. Blotter paper can be obtained from Buckeye Technologies. Memphis. TN. The topsheet material can be obtained from Avgol Nonwoven Industries, Holon, Israel. The fluid intake tester (FIT). of Buckeye design, consists of a top plate and a bottom plate. The top plate is a 29.7 cm x 19.0 cm x 1.3 cm plate of polycarbonate plastic. The plate has a hole cut out of its center and a hollow intake cylinder is mounted in the hole. The inner diameter of the intake cylinder is 2.5 cm and the complete top plate weighs 872 grams. The bottom plate of the FIT is essentially a 29.7 cm x 19.0 cm x 1.3 cm monolithic plate of polycarbonate plastic.

The sample is cut to 7 cm x 20 cm with the longer dimension in the machine direction. The sample weight and thickness are measured and recorded. An "X" is placed at the center of the top of the sample with a marking pen. The sample is centered on the FIT bottom plate. The topsheet is centered on the sample and the FIT top plate is lowered on top of the topsheet. The top plate is centered on the sample so that the intake cylinder is centered on the "X" marked on the sample. A 10-ml insult of fluid is poured into the intake cylinder and the amount of time taken for the sample to acquire the fluid is measured and recorded. This time, reported in seconds (s), is the acquisition time for the sample. Simultaneous with the end of the acquisition time, a 20-minute waiting period begins. At the end of the waiting period, the stain size is measured and recorded lengthwise (machine direction) on both the top and the bottom of the sample. Stain size is reported in millimeters (mm). The polycarbonate plastic is clear so that the stain size can be viewed through the plastic. The bottom stain can be measured by temporarily flipping over the FIT so that the bottom plate faces up. Rewet is measured by removing the top FIT plate. then placing a pre-weighed stack of eight S22 blotter papers on the topsheet of the sample. The foam is placed on the paper and the weight is placed on top of the foam (the weight, the foam and the paper constitute a 3.4 kPa (0.5) psi pressure on the sample) for two minutes. The rewet, reported in grams (g), is calculated by subtracting the initial weight of the stack of papers from the final weight of the stack of papers. This combination test is usually performed in triplicate and the results are averaged.

**Examples A through H: Laboratory Samples for Rewet Measurement**

The invention is illustrated here by performing a series of experiments in which absorbent structures are constructed and tested. The experimental variables are the type of cellulosic fiber used in the wicking layer of the structure, the basis weight of the wicking layer and the utilization of a web treatment on the wicking layer. Other elements in the overall absorbent structure, such as overall density, overall basis weight, composition of acquisition layer and composition of storage layer, are held constant for the purpose of illustrating the advantages of the particular wicking layers of this invention. The term "Unicore" as used herein means a multi-layered absorbent structure which could be manufactured on a continuous forming machine. More specifically, the Unicore structures of this invention have discrete layers for fluid acquisition, storage, and distribution (wicking) which layers are in contact thereby allowing fluid transfer between layers. It is understood that such Unicore structures could also be fabricated from individually prepared layers of material.

In Examples A through H, the top acquisition layer comprises 35 gsm of polyester staple fiber (15 dpf x 6 mm, Grade 376X2, Wellman, Inc., Johnsonville, SC) to which is applied a latex binder (Airflex 192, Air Products and Chemicals, Allentown, PA). The middle layer comprises 90 gsm of HPF fiber (a mercerized Southern softwood fiber available from Buckeye Technologies, Memphis, TN) and about 9 gsm bicomponent binder fiber (Grade AL-Adhesion-C, 1.7 dtex x 6 mm, FiberVisions. Covington. GA). These two layers were made separately on an airlaid pilot machine. Minimal compaction was used in the construction of these two layers. In these examples, the structures all contained the same top and middle layers.

The bottom wicking layers for Examples A through H were made using a laboratory airlaid handsheet apparatus. For the wicking layers, we examined three effects. The first effect was the type of cellulosic fiber used in the wicking layer. Half of the wicking layers were made with Grade ND-416 fiber from Weyerhaeuser Co., Tacoma WA. The other half of the wicking layers were made with bleached kraft eucalyptus fiber from Aracruz Celulose (USA), Raleigh, NC. The wicking layers contained about 10% bicomponent binder fiber by weight. The second effect was the basis weight of the wicking layer, which was fixed at either 50 or 70 gsm of cellulosic. The third effect was the use of a web treatment; half of the samples received the web treatment. The web treatment involved pre-densification of the wicking layer, in which the wicking layer was formed in the handsheet apparatus, then densified in a laboratory press. A piece of forming wire fabric was placed on the bottom platen of the press to impose a pattern of densified regions and less densified regions in the wicking layer. For the wicking layers subjected to the web treatment (Examples E through H), they were pre-densified. on average, to an overall, apparent density of about 0.06 to 0.07 g/cc, with the density in the pattern imposed by the forming wire fabric likely much higher than 0.10 g/cc.

Unicore structures were assembled by hand in the laboratory. The structures in these examples were all densified to an overall, apparent density of 0.09 g/cc. These structures contained identical top and middle layers. Focusing on the wicking layer, we made eight structures using all possible combinations of the three effects. Table 5 shows test data for the eight structures, Examples A through H. Figure 5 is a graph of rewet data for these examples. Examples A through H can be divided into four head-to-head comparisons of eucalyptus and ND-416. These comparisons are indicated by the different textures used for the bars in Figure 5. In each comparison, eucalyptus fiber provides for lower rewet compared to ND-416. A statistical analysis of the data outlined in Table 5 shows that all of the effects of this laboratory study significantly influenced rewet. The ranking of the relative strength of the effects is web treatment (with is better than without) < basis weight (higher is better than lower) << choice of fiber (eucalyptus is much better than ND-416).

Table 5.

| Results for laboratory evaluation of eucalyptus fiber in Unicore. | | | | | | | | | | | | | | | | | |
|---|---|---|---|---|---|---|---|---|---|---|---|---|---|---|---|
| Example | Pulp | Layer basis weight $g/m^2$ | Web treatment | Acquisition *s* | | Rewet *g* | | Bottom stain size *mm* | | Overall basis weight $g/m^2$ | | Density *g/cc* | |
| | | | | Ave | SD | Ave | SD | Ave | SD | Ave | SD | Ave | SD |
| A | Eucalyp. | 50 | No | 9.62 | 0.84 | 1.65 | 0.18 | 158 | 5 | 210 | 4 | 0.091 | 0.002 |
| B | ND-416 | 50 | No | 9.10 | 0.66 | 2.46 | 0.25 | 134 | 4 | 210 | 3 | 0.091 | 0.002 |
| | | | | | | | | | | | | | |
| C | Eucalyp. | 70 | No | 8.96 | 1.02 | 1.32 | 0.20 | 140 | 9 | 233 | 9 | 0.091 | 0.003 |
| D | ND-416 | 70 | No | 8.83 | 0.24 | 2.37 | 0.27 | 133 | 6 | 230 | 9 | 0.092 | 0.004 |
| | | | | | | | | | | | | | |
| E | Eucalyp. | 50 | Yes | 8.74 | 0.53 | 1.63 | 0.56 | 158 | 6 | 209 | 3 | 0.090 | 0.003 |
| F | ND-416 | 50 | Yes | 9.53 | 0.65 | 2.20 | 0.24 | 168 | 4 | 208 | 5 | 0.092 | 0.005 |
| | | | | | | | | | | | | | |
| G | Eucalyp. | 70 | Yes | 10.66 | 1.44 | 0.83 | 0.31 | 167 | 6 | 229 | 2 | 0.100 | 0.002 |
| H | ND-416 | 70 | Yes | 9.07 | 0.69 | 1.78 | 0.46 | 148 | 6 | 231 | 4 | 0.097 | 0.002 |

**Examples I through K: Airlaid Pilot Samples for Rewet Measurements**

Examples I through K have the same top and middle layers. The top layer comprises 35 gsm of Wellman PET with about 6 gsm of Airflex 192 latex for bonding. The middle layer comprises 70 gsm HPF fiber, 56 gsm Favor 1180 superabsorbent powder (Stockhausen, Inc., Greensboro, NC) and about 9.5 gsm FiberVisions bicomponent fiber, AL-Adhesion-C, 1.7 dtex x 4 mm, The bottom layer comprises 70 gsm of cellulosic fiber and about 5.3 gsm of the same FiberVisions bicomponent fiber. Examples I through K were made as unitary structures on a 0.6 meter-wide, three-head pilot line. Example I was made with ND-416 fiber and about 2.5 gsm of Airflex 192 latex was applied to the wire side of the web to control dusting. Example J was made identically to Example I, except that eucalyptus fiber was substituted for ND-416 fiber and a tissue carrier (18 gsm, Cellu Tissue Co., East Hartford, CT) was substituted for the wire-side latex. Example K was made identically to J, except that the web treatment was used for the eucalyptus (bottom) layer.

Table 6 shows test data for three samples, Examples I through K. Note the basis weight difference between Example I and Examples J and K. This can be attributed to the carrier tissue. which imparts essentially no performance advantages to the samples. Figure 6 shows rewet results for Examples I through K. Confirming the laboratory work, the pilot samples show that eucalyptus and the web treatment both help to improve rewet.

Table 6.

| Rewet Results for Pilot Plant Samples | | | | | | | | | | | | | |
|---|---|---|---|---|---|---|---|---|---|---|---|---|---|
| Example | Pulp | Layer basis weight *g*/*m*$^2$ | Web treatment | Acquisition *s* | | Rewet *g* | | Bottom stain size *mm* | | Overall basis weight *g*/*m*$^2$ | | Density *g*/*cc* | |
| | | | | Ave | SD | Ave | SD | Ave | SD | Ave | SD | Ave | SD |
| 1 | ND-416 | 70 | No | 15.29 | 2.55 | 2.81 | 0.60 | 99 | 9 | 250 | 7 | 0.086 | 0.0001 |
| | | | | | | | | | | | | | |
| J | Eucalyp. | 70 | No | 13.54 | 2.03 | 1.11 | 0.24 | 123 | 9 | 277 | 19 | 0.088 | 0.007 |
| K | Eucalyp. | 70 | Yes | 9.99 | 1.54 | 0.42 | 0.04 | 144 | 4 | 270 | 7 | 0.086 | 0.003 |

**Examples L through W: survey of hardwood pulps**

In these examples, the experimental variables are the type of hardwood fiber used in the wicking layer of the structure and the basis weight of the wicking layer. An example using the softwood fiber ND-416 in the wicking layer is included for comparison purposes. Other elements of the overall absorbent structure, such as overall density, overall basis weight, composition of acquisition layer and composition of storage layer, are held constant for the purpose of illustrating the advantages of the particular wicking layers of the present invention.

In Examples L through W, the top acquisition layer comprises 35 gsm of polyester staple fiber (15 dpf x 6 mm, Grade 376X2, Wellman, Inc., Johnsonville, SC) and about 6 gsm of latex binder (Airflex 192. Air Products and Chemicals, Allentown. PA). The middle layer comprises 60 gsm of HPF fiber (a mercerized Southern softwood fiber available from Buckeye Technologies, Memphis, TN) and about 7 gsm bicomponent binder fiber (Grade AL-Adhesion-C, 1.7 dtex x 4 mm, FiberVisions, Covington, GA). These two layers were made separately on an airlaid pilot machine. Minimal compaction was used in the construction of these two layers. In these examples. the structures all contained the same top and middle layers.

The bottom wicking layers for Examples L through W were made using a laboratory airlaid handsheet apparatus. For these examples, we examined two effects. The first effect was the type of cellulosic fiber used in the wicking layer. Six cellulosic fibers were used, five hardwoods and one softwood:

**Hardwoods**

- Bleached kraft eucalyptus pulp from Aracruz Celulose (USA), Raleigh, NC (Aracruz)
- Dissolving eucalyptus pulp, Solucell-400. from Klabin Bacell, Camaçari, Brazil (Solucell)
- Bleached kraft eucalyptus pulp, Primacell, from Riocell, Guaiba, Brazil (Primacell)
- Bleached kraft birch pulp from Kaukas Mill, UPM-Kymmene, Finland (Birch)
- Dissolving eucalyptus pulp from Sappi Saiccor, Johannesburg, South Africa (Saiccor)

**Softwood**

- Bleached Southern softwood kraft pulp, Grade ND-416, from Weyerhaeuser Co., Tacoma, WA (ND-416)

The wicking layers contained about 10% bicomponent binder fiber by weight (Grade AL-Adhesion-C, 1.7 dtex x 4 mm, FiberVisions, Covington, GA). The second effect examined was the basis weight of the wicking layer, which was fixed at either 50 or 80 gsm of cellulosic. Unicore structures were assembled in the laboratory. The structures in Examples L through W were densified to an overall, apparent density target of 0.085 g/cc. Twelve structures were made (six pulps at two basis weights). Testing was performed in triplicate using the methods described in detail in the 4/4/00 disclosure.

Table 1 shows test data for Examples L through W. Figure 1 is a graph of rewet data for these examples. Rewet results for both basis weights, 50 gsm and 80 gsm. are plotted in Figure 1. At each basis weight, the hardwood pulps generally provide for lower rewet compared to the softwood pulp.

Figure 8 is a plot of bottom stain size data for these examples. Although there is some scatter in the data, at each basis weight, the bottom stain size for the softwood Pulp is generally shorter than the bottom stain size for the hardwood pulps. The hardwood pulps are more successful at wicking the fluid away from the insult site.

Concerning the relation between rewet and bottom stain size, we hypothesize that improved wicking (a larger bottom stain size) results in enhanced fluid retention (as measured by rewet) by moving fluid away from relatively saturated pans of the structure (the insult site) to less saturated parts of the structure (away from the insult site). Thus, a structure with exceptional wicking would be capable of literally sucking fluid away from the insult site. Such a structure could be described as a "high suction" core.

Table 7.

| Results for laboratory evaluation of various pulps as wicking lavers in Unicore | | | | | | | | | | | | | | |
|---|---|---|---|---|---|---|---|---|---|---|---|---|---|---|
| Example | Pulp | Laver basis weight $g/m^2$ | Acquisition *s* | | Rewet *g* | | Bottom stain size *mm* | | Overall basis weight $g/m^2$ | | Density *g/cc* | |
| | | | Ave | SD | Ave | SD | Ave | SD | Ave | SD | Ave | SD |
| L | Aracruz | 50 | 10.24 | 0.89 | 1.95 | 0.37 | 189 | 5 | 165 | 8 | 0.086 | 0.007 |
| M | Aracruz | 80 | 8.03 | 0.46 | 0.88 | 0.13 | 165 | 6 | 195 | 5 | 0.080 | 0.003 |
| | | | | | | | | | | | | |
| N | Solucell | 50 | 9.39 | 0.76 | 1.69 | 0.33 | 199 | 2 | 163 | 3 | 0.085 | 0.002 |
| O | Solucell | 80 | 7.95 | 0.66 | 0.67 | 0.12 | 175 | 7 | 201 | 16 | 0.084 | 0.001 |
| | | | | | | | | | | | | |
| P | Primacell | 50 | 10.21 | 0.97 | 2.02 | 0.21 | 193 | 4 | 155 | 11 | 0.079 | 0.005 |
| Q | Primacell | 80 | 8.82 | 1.68 | 1.42 | 0.52 | 172 | 1 | 192 | 10 | 0.083 | 0.003 |
| | | | | | | | | | | | | |
| R | Birch | 50 | 9.64 | 1.80 | 2.32 | 0.35 | 186 | 10 | 156 | S | 0.080 | 0.005 |
| S | Birch | 80 | 9.36 | 1.57 | 2.57 | 0.15 | 158 | 7 | 185 | 9 | 0.086 | 0.007 |
| | | | | | | | | | | | | |
| T | Saiccor | 50 | 9.76 | 1.86 | 2.08 | 0.13 | 191 | 4 | 155 | 5 | 0.078 | 0.004 |
| U | Saiccor | 80 | 7.31 | 0.87 | 1.19 | 0.29 | 170 | 9 | 186 | 2 | 0.077 | 0.002 |
| | | | | | | | | | | | | |
| V | ND-416 | 50 | 8.62 | 0.55 | 2.94 | 0.30 | 177 | 2 | 153 | 1 | 0.078 | 0.002 |
| W | ND-416 | 80 | 6.96 | 0.27 | 3.36 | 0.21 | 160 | 20 | 170 | 10 | 0.070 | 0.004 |

**Claims**

**1.** An absorbent core (15) comprising:

(1) an acquisition layer (11),

(2) a storage layer (12) having absorbent capacity disposed beneath and in fluid communication with the acquisition layer (11). the storage layer (12) comprising superabsorbent materials and fibers; and

(3) a wicking layer (13) disposed beneath and in fluid communication with the storage layer (12), comprising compressible hardwood pulp having a density of between about 0.05 and about 0.4 g/cc, where the ratio of the vertical wicking height of the wicking layer to the vertical wicking height of the storage layer is equal to or greater than 1.25.

**2.** The absorbent core (15) according to claim 1, wherein the wicking layer (13) comprises from about 50 percent by weight to about 99.9 percent by weight of hardwood fibers and from about 0.1 percent by weight to about 50 percent by weight synthetic fibers, the storage layer (12) comprises synthetic fibers, chemically treated cellulosic fibers, wood pulp, superabsorbents, and combinations thereof, and has a density of between 0.05 and 0.25 g/cc, and the acquisition layer has a density of between 0.04 to 0.1 g/cc.

**3.** The absorbent core (15) according to claims 1 or 2, wherein the ratio of vertical wicking height of the wicking layer (13) to the vertical wicking height of the storage layer (12) is equal to or greater than 3.0.

**4.** The absorbent core (15) according to anyone of the claims 1-3, wherein the compressible hardwood pulp is selected from the group consisting of eucalyptus, birch, aspen, maple, cotton wood, willow, oak, beech, poplar, basswood and combinations thereof.

**5.** The absorbent core (15) according to claim 4, wherein the compressible hardwood pulp is eucalyptus.

**6.** The absorbent core (15) according to anyone of claims 1-5, wherein the wicking layer (13) further comprises chemically treated softwood pulp fibers.

**7.** The absorbent core (15) according to anyone of claims 1-6. wherein the wicking layer (13) is imprinted with a compression pattern.

**8.** The absorbent core (15) according to anyone of claims 1-7, wherein the wicking layer (13) has a density of between 0.1 and 0.3 g/cc.

**9.** The absorbent core (15) according to anyone of claims 1-8, wherein the core has a rewet value of about 3.0 g or less, preferably about 2.0 g or less, and more preferably about 1.0 g or less.

**10.** The absorbent core (15) according to anyone of claims 1-9, wherein the absorbent core is a unitary absorbent core produced in a series of unit operations in a continuous process.

**11.** An absorbent core (15) according to anyone of claims 1-10, wherein the wicking layer (13) is imprinted to impose a pattern of densified regions and less densified regions.

**12.** An absorbent article comprising:

(A) a liquid permeable top sheet (10),

(B) a liquid impermeable back sheet (14), and

(C) an absorbent core (15) according to anyone of claims 1-11, disposed between the topsheet (10) and the backsheet (14),

wherein an acquisition layer (11) of said absorbent core (15) is disposed beneath and in fluid communication with the top sheet (10).

**13.** The absorbent article according to claim 12, wherein the article is selected from the group consisting of infant diapers, training pants, adult incontinence briefs, feminine hygiene pads, surgical drapes and wound dressings.

**14.** A method of making an absorbent core (15) according to anyone of claims 1- 11 comprising:

(A) forming a wicking layer (13) comprising compressible hardwood pulp fibers;

(B) compressing the wicking layer to a density of between 0.05 and 0.4 g/cc;

(C) forming a storage layer (12) having absorbent capacity and in fluid communication with the wicking layer, the storage layer comprising superabsorbent materials and fibers; and

(D) forming an acquisition layer (11) in fluid communication with the storage layer (12),

where the ratio of the vertical wicking height of the wicking layer to the vertical wicking height of the storage layer is equal to or greater than 1.25.

**15.** The method according to claim 14, wherein the ratio of vertical wicking height of the wicking layer (13) to the vertical wicking height of the storage layer (12) is equal to or greater than 3.0.

**16.** The method according to claim 14 or 15, wherein the compressible hardwood pulp is selected from the group consisting of eucalyptus, birch, aspen, maple, cotton wood, willow, oak, beech, poplar, basswood and combinations thereof.

**17.** The method according to claim 16, wherein the compressible hardwood pulp is eucalyptus.

**18.** The method according to anyone of claims 14-17, wherein the wicking layer (13) further comprises chemically treated softwood pulp fibers.

**19.** The method according to anyone of claims 14-18, wherein the wicking layer (13) is imprinted with a compression pattern.

**20.** The method according to claim 19, wherein said compression pattern comprises a pattern of densified and less densified regions, imprinted on said wicking layer (13) during compression between a forming or transfer fabric and a compaction roll.

**21.** The method according to claim 19, wherein the compression pattern is imprinted on said wicking layer (13) during compression between a patterned compaction roll and a smooth roll, or between two patterned compaction rolls.

**22.** The method according to anyone of claims 14-21, wherein the core has a rewet value of about 3.0 g or less, preferably about 2.0 g or less, and more preferably about 1.0 g or less.

**23.** The method according to anyone of claims 14-22, wherein the absorbent core (15) is a unitary absorbent core produced in a series of unit operations in a continuous process.

**24.** The method according to claim 23, wherein the process is an airlaid process.

**25.** A method of making the absorbent article of claim 12 comprising:

(A) providing a liquid impervious back sheet (14);

(B) forming a wicking layer (13) comprising compressible hardwood pulp fibers;

(C) compressing the wicking layer to a density of between 0.05 and 0.4 g/cc;

(D) forming a storage layer (12) having absorbent capacity and in fluid communication with the wicking layer, the storage layer comprising superabsorbent materials and fibers;

(E) forming an acquisition layer (11) in fluid communication with the storage layer; and

(F) providing a liquid pervious top sheet (10) in fluid communication with the acquisition layer;

where the ratio of the vertical wicking height of the wicking layer to the vertical wicking height of the storage layer is equal to or greater than 1.25.

**26.** The method according to claim 25, wherein the wicking layer (13) further comprises chemically treated softwood pulp fibers.

**Patentansprüche**

**1.** Ein absorbierender Kern (15), der aufweist:

(1) eine Aufnahmeschicht (11);
(2) eine darunterliegend angeordnete und im Flüssigkeitsaustausch mit der Aufnahmeschicht (11 ) stehende Lagerungsschicht (12) mit einer Saugkapazität, wobei die Lagerungsschicht (12) hochabsorbierende Materialien und Fasern aufweist; und
(3) eine darunterliegend angeordnete und im Flüssigkeitsaustausch mit der Lagerungsschicht (12) stehende kapillare Schicht (13), die zusammenpressbare Hartholzpülpe mit einer Dichte zwischen ungefähr 0,05 und ungefähr 0,4 g/cm3 aufweist,

wobei das Verhältnis der vertikalen Kapillarhöhe der kapillaren Schicht zu der vertikalen Kapillarhöhe der Lagerungsschicht gleich oder größer als 1,25 ist.

2. Der absorbierende Kern (15) nach Anspruch 1, wobei die kapillare Schicht (13) ungefähr 50% an Gewicht bis ungefähr 99,9% an Gewicht Hartholzfasern und ungefähr 0,1% an Gewicht bis ungefähr 50% an Gewicht synthetische Fasern aufweist, die Lagerungsschicht (12) synthetische Fasern, chemisch behandelte Zellulosefasern, Holzpülpe, hochabsorbierendes Material und Kombinationen davon aufweist und eine Dichte zwischen 0,05 und 0,25 g/cm3 besitzt, und die Aufnahmeschicht eine Dichte zwischen 0,04 bis 0,1 g/cm3 besitzt.

3. Der absorbierende Kern (15) nach Anspruch 1 oder 2, wobei das Verhältnis der vertikalen Kapillarhöhe der kapillaren Schicht (13) zu der vertikalen Kapillarhöhe der Lagerungsschicht (12) gleich oder größer als 3,0 ist.

4. Der absorbierende Kern (15) nach einem der Ansprüche 1 bis 3, wobei die zusammenpressbare Hartholzpülpe aus der aus Eukalyptus, Birke, Espe, Ahorn, Baumwollholz, Weide, Eiche, Buche, Pappel, Baßholz und aus den Kombinationen derselben gebildeten Gruppe gewählt wird.

5. Der absorbierende Kern (15) nach Anspruch 4, wobei die zusammenpressbare Hartholzpülpe Eukalyptus ist.

6. Der absorbierende Kern (15) nach einem der Ansprüche 1 bis 5, wobei die kapillare Schicht (13) außerdem chemisch behandelte Weichholzpülpefasern aufweist.

7. Der absorbierende Kern (15) nach einem der Ansprüche 1 bis 6, wobei die kapillare Schicht (13) mit einem Druckmuster bedruckt ist.

8. Der absorbierende Kern (15) nach einem der Ansprüche 1 bis 7, wobei die kapillare Schicht (13) eine Dichte zwischen 0,1 und 0,3 g/cm3 besitzt.

9. Der absorbierende Kern (15) nach einem der Ansprüche 1 bis 8, wobei der Kern einen Rückfeuchtewert von ungefähr 3,0 g oder weniger besitzt, vorteilhafterweise ungefähr 2,0 g oder weniger, und vorzugsweise ungefähr 1,0 g oder weniger.

10. Der absorbierende Kern (15) nach einem der Ansprüche 1 bis 9, wobei der absorbierende Kern ein einheitlicher absorbierender Kern ist, der in einer Reihe von einheitlichen Vorgängen in einem kontinuierlichen Verfahren produziert wurde.

11. Ein absorbierender Kern (15) nach einem der Ansprüche 1 bis 10, wobei die kapillare Schicht (13) bedruckt ist, um ein Muster aus verdichteten Regionen und weniger verdichteten Regionen aufzuprägen.

12. Ein absorbierender Artikel, der aufweist:

(A) ein flüssigkeitsdurchlässiges oberes Blatt (10),
(B) ein flüssigkeitsundurchlässiges rückseitiges Blatt (14), und
(C) einen absorbierenden Kern (15) nach einem der Ansprüche 1 bis 11, der zwischen dem oberen Blatt (10) und dem rückseitigen Blatt (14) angeordnet ist,

wobei eine Aufnahmeschicht (11) des besagten absorbierenden Kerns (15) unterhalb und im Flüssigkeitsaustausch mit dem oberen Blatt (10) angeordnet ist.

13. Der absorbierende Artikel nach Anspruch 12 , wobei der Artikel aus der aus Säuglingswindeln, Trainingshosen, Hosen für Erwachseneninkontinenz, Binden für weibliche Hygiene, chirurgischen Garnituren und Verbänden für Verletzungen gebildeten Gruppe gewählt wird.

14. Ein Verfahren zur Herstellung eines absorbierenden Kerns (15) nach einem der Ansprüche 1 bis 11, aufweisend:

(A) eine kapillare Schicht (13) zu bilden, die zusammenpressbare Hartholzpülpefasern aufweist;
(B) die kapillare Schicht bis zu einer Dichte zwischen 0,05 und 0,4 g/cm3 zu verdichten;
(C) eine eine Saugkapazität besitzende und im Flüssigkeitsaustausch mit der kapillaren Schicht stehende Lagerungsschicht (12) zu bilden, wobei die Lagerungsschicht hochabsorbierende Materialien und Fasern aufweist; und

(D) eine im Flüssigkeitsaustausch mit der Lagerungsschicht (12) stehende Aufnahmeschicht (11) zu bilden,

wobei das Verhältnis der vertikalen Kapillarhöhe der kapillaren Schicht zu der vertikalen Kapillarhöhe der Lagerungsschicht gleich oder größer als 1,25 ist.

**15.** Das Verfahren nach Anspruch 14, wobei das Verhältnis der vertikalen Kapillarhöhe der kapillaren Schicht (13) zu der vertikalen Kapillarhöhe der Lagerungsschicht (12) gleich oder größer als 3,0 ist.

**16.** Das Verfahren nach Anspruch 14 oder 15, wobei die zusammenpressbare Hartholzpülpe aus der aus Eukalyptus, Birke, Espe, Ahorn, Baumwollholz, Weide, Eiche, Buche, Pappel, Baßholz und aus den Kombinationen derselben gebildeten Gruppe gewählt wird.

**17.** Das Verfahren nach Anspruch 16, wobei die zusammenpressbare Hartholzpülpe Eukalyptus ist.

**18.** Das Verfahren nach einem der Ansprüche 14 bis 17, wobei die kapillare Schicht (13) außerdem chemisch behandelte Weichholzpülpefasern aufweist.

**19.** Das Verfahren nach einem der Ansprüche 14 bis 18, wobei die kapillare Schicht (13) mit einem Druckmuster bedruckt ist.

**20.** Das Verfahren nach Anspruch 19, wobei besagtes Druckmuster ein Muster aus verdichteten und weniger verdichteten Regionen aufweist, das während dem Zusammenpressen zwischen einem Formungs- oder Übertragungsstoff und einer Verdichtungsrolle auf besagter kapillarer Schicht (13) gedruckt wird.

**21.** Das Verfahren nach Anspruch 19, wobei das Druckmuster auf besagter kapillarer Schicht (13) während des Zusammenpressens zwischen einer gemusterten Verdichtungsrolle und einer glatten Rolle oder zwischen zwei gemusterten Verdichtungsrollen gedruckt wird.

**22.** Das Verfahren nach einem der Ansprüche 14 bis 21, wobei der Kern einen Rückfeuchtewert von ungefähr 3,0 g oder weniger besitzt, vorteilhafterweise ungefähr 2,0 g oder weniger, und vorzugsweise ungefähr 1,0 g oder weniger.

**23.** Das Verfahren nach einem der Ansprüche 14 bis 22, wobei der absorbierende Kern (15) ein einheitlicher absorbierender Kern ist, der in einer Reihe von einheitlichen Vorgängen in einem kontinuierlichen Verfahren produziert wurde.

**24.** Das Verfahren nach Anspruch 23, wobei das Verfahren ein Luftträgerverfahren ist.

**25.** Ein Verfahren zur Herstellung des absorbierenden Artikels des Anspruchs 12, aufweisend:

(A) ein flüssigkeitsundurchlässiges rückseitiges Blatt (14) zur Verfügung zu stellen;
(B) eine kapillare Schicht (13) zu bilden, die zusammenpressbare Hartholzpülpefasem aufweist;
(C) die kapillare Schicht bis zu einer Dichte zwischen 0,05 und 0,4 g/cm3 zu verdichten;
(D) eine eine Saugkapazität besitzende und im Flüssigkeitsaustausch mit der kapillaren Schicht stehende Lagerungsschicht (12) zu bilden, wobei die Lagerungsschicht hochabsorbierende Materialien und Fasern aufweist;
(E) eine im Flüssigkeitsaustausch mit der Lagerungsschicht stehende Aufnahmeschicht (11) zu bilden; und
(F) ein flüssigkeitsdurchlässiges oberes Blatt (10) zur Verfügung zu stellen, das im Flüssigkeitsaustausch mit der Aufnahmeschicht steht;

wobei das Verhältnis der vertikalen Kapillarhöhe der kapillaren Schicht zu der vertikalen Kapillarhöhe der Lagerungsschicht gleich oder größer als 1,25 ist.

**26.** Das Verfahren nach Anspruch 25, wobei die kapillare Schicht (13) außerdem chemisch behandelte Weichholzpülpefasern aufweist.

## Revendications

1. Un noyau absorbant (15) comportant :

   (1) une couche d'acquisition (11) ;
   (2) une couche de stockage (12) ayant une capacité absorbante, disposée en dessous, et en communication de fluide avec la couche d'acquisition (11), la couche de stockage (12) comportant des fibres et des matériaux superabsorbants ; et
   (3) une couche capillaire (13) disposée en dessous et en communication de fluide avec la couche de stockage (12), comportant de la pulpe de bois dure compressible ayant une densité entre environ 0,05 et environ 0,4 $g/cm^3$, où la hauteur capillaire verticale de la couche capillaire à la hauteur capillaire verticale de la couche de stockage est égale ou supérieure à 1,25.

2. Le noyau absorbant (15) selon la revendication 1, dans lequel la couche capillaire (13) comporte d'environ 50 % en poids à environ 99,9 % en poids de fibres de bois dur et d'environ 0,1 % en poids à environ 50 % en poids de fibres synthétiques, la couche de stockage (12) comporte des fibres synthétiques, des fibres de cellulose chimiquement traitées, de la pulpe de bois, des superabsorbants et des combinaisons de ceux-ci, et présente une densité d'entre 0,05 et 0,25 $g/cm^3$, et la couche d'acquisition possède une densité d'entre 0,04 à 0,1 $g/cm^3$.

3. Le noyau absorbant (15) selon les revendications 1 ou 2, dans lequel le rapport de la hauteur capillaire verticale de la couche capillaire (13) à la hauteur capillaire verticale de la couche de stockage (12) est égale ou supérieure à 3,0.

4. Le noyau absorbant (15) selon l'une quelconque des revendications 1-3, dans lequel la pulpe de bois dur compressible est choisie dans le groupe constitué de l'eucalyptus, du bouleau, du tremble, de l'érable, du peuplier du Canada, du saule, du chêne, du hêtre, du peuplier, du peuplier d'Amérique et des combinaisons de ceux-ci.

5. Le noyau absorbant (15) selon la revendication 4, dans lequel la pulpe de bois dur compressible est de l'eucalyptus.

6. Le noyau absorbant (15) selon l'une quelconque des revendications 1 à 5, dans lequel la couche capillaire (13) comporte en outre des fibres en pulpe de bois tendre, chimiquement traitées.

7. Le noyau absorbant (15) selon l'une quelconque des revendications 1 à 6, dans lequel la couche capillaire (13) est imprimée au moyen d'un modèle pour compression.

8. Le noyau absorbant (15) selon l'une quelconque des revendications 1 à 7, dans lequel la couche capillaire (13) présente une densité entre 0,1 et 0, 3 $g/cm^3$.

9. Le noyau absorbant (15) selon l'une quelconque des revendications 1 à 8, dans lequel le noyau présente une valeur de réhumidification d'environ 3,0 g ou moins, de préférence d'environ 2,0 g ou moins, et plus avantageusement d'environ 1,0 g ou moins.

10. Le noyau absorbant (15) selon l'une quelconque des revendications 1 à 9, dans lequel le noyau absorbant est un noyau absorbant unitaire produit dans une série de traitements unitaires dans un procédé continu.

11. Un noyau absorbant (15) selon l'une quelconque des revendications 1 à 10, dans lequel la couche capillaire (13) est imprimée pour imposer un modèle de régions densifiées et de régions moins densifiées.

12. Un article absorbant comportant :

    (A) une feuille supérieure perméable au liquide (10),
    (B) une couche arrière imperméable au liquide (14), et
    (C) un noyau absorbant (15) selon l'une quelconque des revendications 1 à 11, disposé entre la feuille supérieure (10) et la feuille arrière (14),

    dans lequel une couche d'acquisition (11) dudit noyau absorbant (15) est disposée en dessous et en communication de fluide avec la feuille supérieure (10).

**13.** L'article absorbant selon la revendication 12, dans lequel l'article est choisi dans le groupe constitué de couches pour enfant, de queues d'entraînement, de tampons pour incontinance des adultes, de serviettes d'hygiène féminine, de garnitures chirurgicales et de pansements pour blessures.

**14.** Un procédé pour fabriquer un noyau absorbant (15) selon l'une quelconque des revendications 1-11 consistant à :

(A) former une couche capillaire (13) comportant des fibres de pulpe de bois dur compressible ;
(B) comprimer la couche capillaire jusqu'à une densité d'entre 0,05 et 0,4 g/$cm^3$ ;
(C) former une couche de stockage (12) ayant une capacité absorbante et en communication de fluide avec la couche capillaire, la couche de stockage comportant des fibres et des matériaux superabsorbants ; et
(D) former une couche d'acquisition (11) en communication de fluide avec la couche de stockage (12),

où le rapport de la hauteur capillaire verticale de la couche capillaire à la hauteur capillaire verticale de la couche de stockage est égale ou supérieure à 1,25.

**15.** Le procédé selon la revendication 14, dans lequel le rapport de la hauteur capillaire verticale de la couche capillaire (13) à la hauteur capillaire verticale de la couche de stockage (12) est égale ou supérieure à 3,0.

**16.** Le procédé selon la revendication 14 ou 15, dans lequel la pulpe de bois dur compressible est choisie dans le groupe constitué de l'eucalyptus, du bouleau, du tremble, de l'érable, du peuplier du Canada, du saule, du chêne, du hêtre, du peuplier, du peuplier d'Amérique et des combinaisons de ceux-ci.

**17.** Le procédé selon la revendication 16, dans lequel la pulpe de bois dur compressible est de l'eucalyptus.

**18.** Le procédé selon l'une quelconque des revendications 14-17, dans lequel la couche capillaire (13) comporte en outre des fibres en pulpe de bois tendre, chimiquement traitées.

**19.** Le procédé selon l'une quelconque des revendications 14-18, dans lequel la couche capillaire (13) est imprimée au moyen d'un modèle pour compression.

**20.** Le procédé selon la revendication 19, dans lequel ledit modèle de compression comporte un modèle de régions densifiées et moins densifiées, imprimé sur ladite couche capillaire (13) pendant la compression entre un tissu de formage ou de transfert et un cylindre de compacité.

**21.** Le procédé selon la revendication 19, dans lequel le modèle de compression est imprimé sur ladite couche capillaire (13) pendant la compression entre un rouleau de compacité modélisé et un cylindre doux, ou entre deux cylindres de compacité modélisés.

**22.** Le procédé selon l'une quelconque des revendications 14-21, dans lequel le noyau présente une valeur de réhumidification d'environ 3,0 g ou moins, de préférence environ 2,0 g ou moins, et avantageusement d'environ 1,0 g ou moins.

**23.** Le procédé selon l'une quelconque des revendications 14-22, dans lequel le noyau absorbant (15) est un noyau absorbant unitaire produit dans une série de traitements unitaires dans un procédé en continu.

**24.** Le procédé selon la revendication 23, dans lequel le procédé est un procédé à support d'air.

**25.** Un procédé de fabrication de l'article absorbant de la revendication 12 consistant à :

(A) se procurer une feuille arrière imperméable (14) ;
(B) former une couche capillaire (13) comportant des fibres de bois dur compressible ;
(C) comprimer la couche capillaire jusqu'à une densité d'entre 0,05 et 0,4 g/$cm^3$ ;
(D) former une couche de stockage (12) ayant une capacité absorbante et en communication de fluide avec la couche capillaire, la couche de stockage comportant des fibres et des matériaux superabsorbants ;
(E) former une couche d'acquisition (11) en communication de fluide avec la couche de stockage ; et
(F) fournir une feuille supérieure perméable au liquide (10) en communication de fluide avec la couche d'acquisition ;

où le rapport de la hauteur capillaire verticale de la couche capillaire à la hauteur capillaire verticale de la couche de stockage est égale ou supérieure à 1,25.

**26.** Le procédé selon la revendication 25, dans lequel la couche capillaire (13) comporte en outre des fibres de pulpe de bois tendre, chimiquement traitées.

FIG. 1

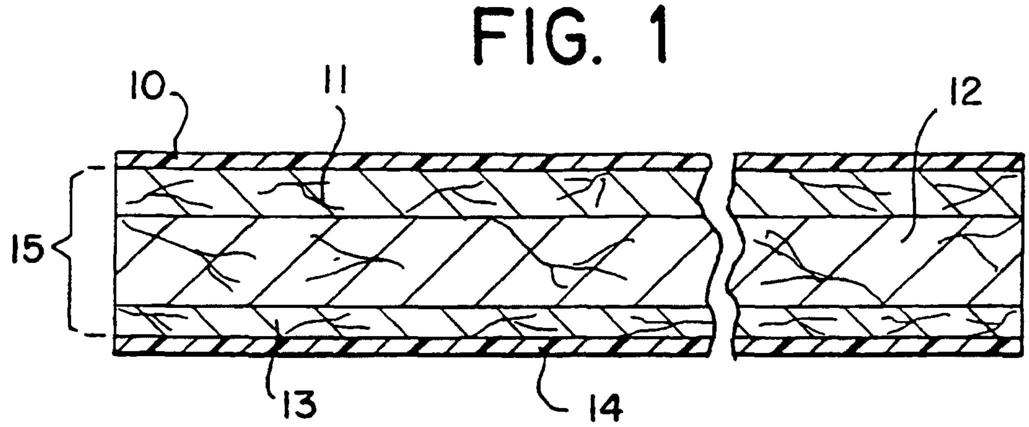


FIG. 2

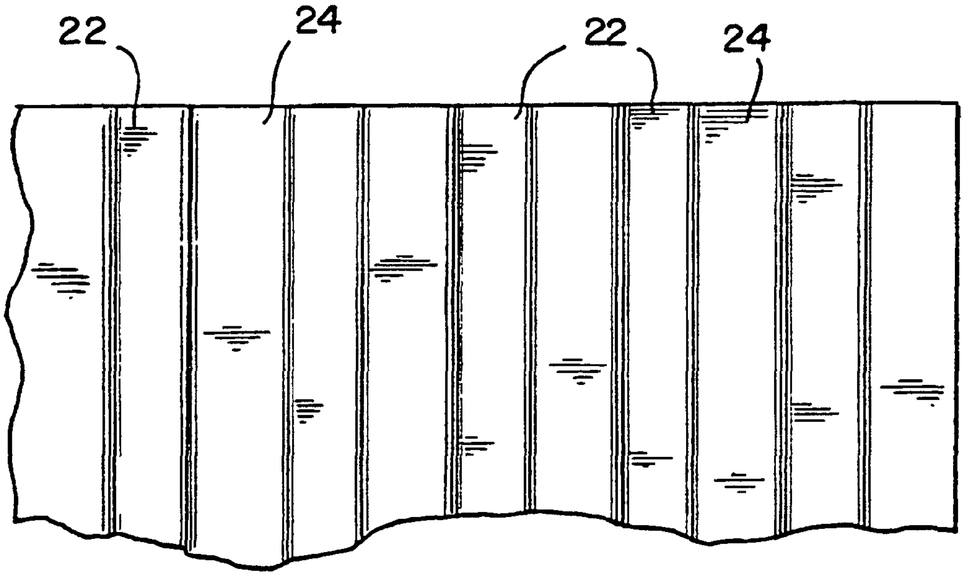


FIG. 3

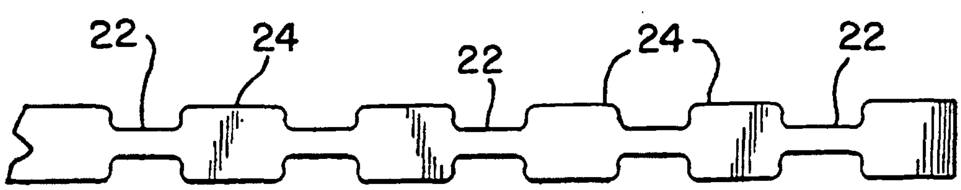

FIG. 4

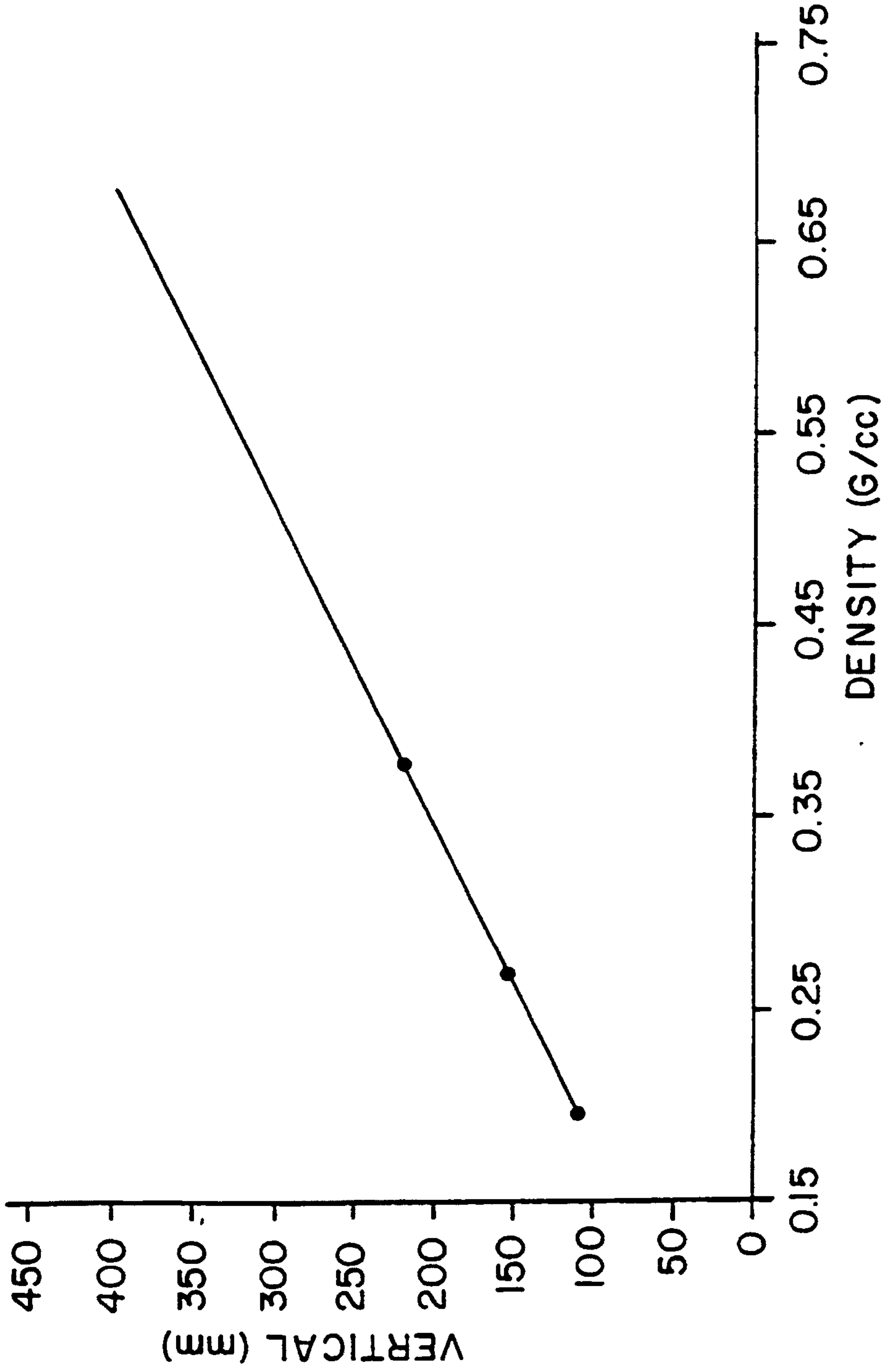
VERTICAL (mm)
0
50
100
150
200
250
300
350
400
450
DENSITY (G/cc)
0.15
0.25
0.35
0.45
0.55
0.65
0.75

FIG. 5

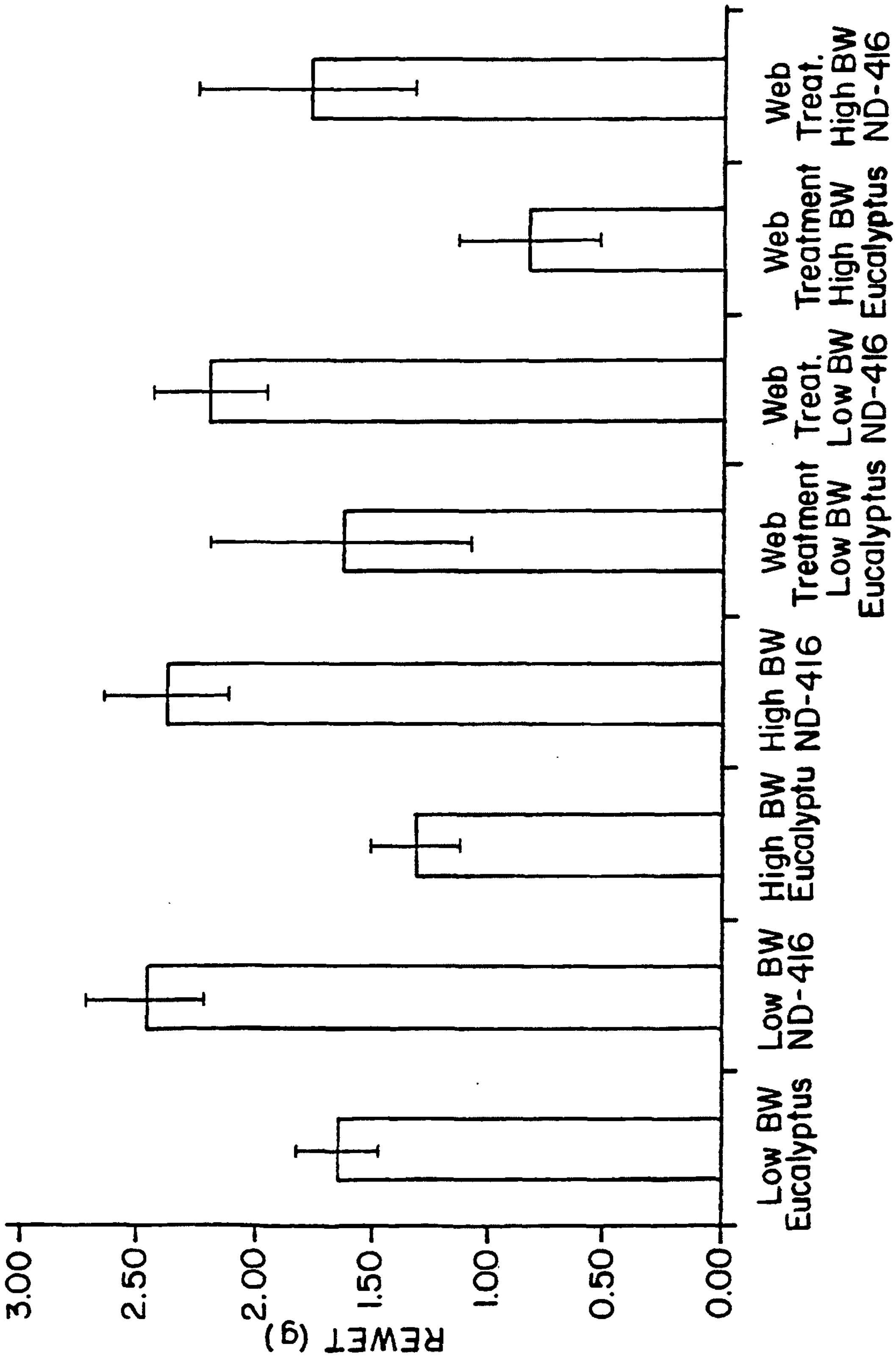
REWET (g)
3.00
2.50
2.00
1.50
1.00
0.50
0.00
Low BW Eucalyptus
Low BW ND-416
High BW Eucalyptu
High BW ND-416
Web Treatment Low BW Eucalyptus
Web Treat. Low BW ND-416
Web Treatment High BW Eucalyptus
Web Treat. High BW ND-416

FIG. 6

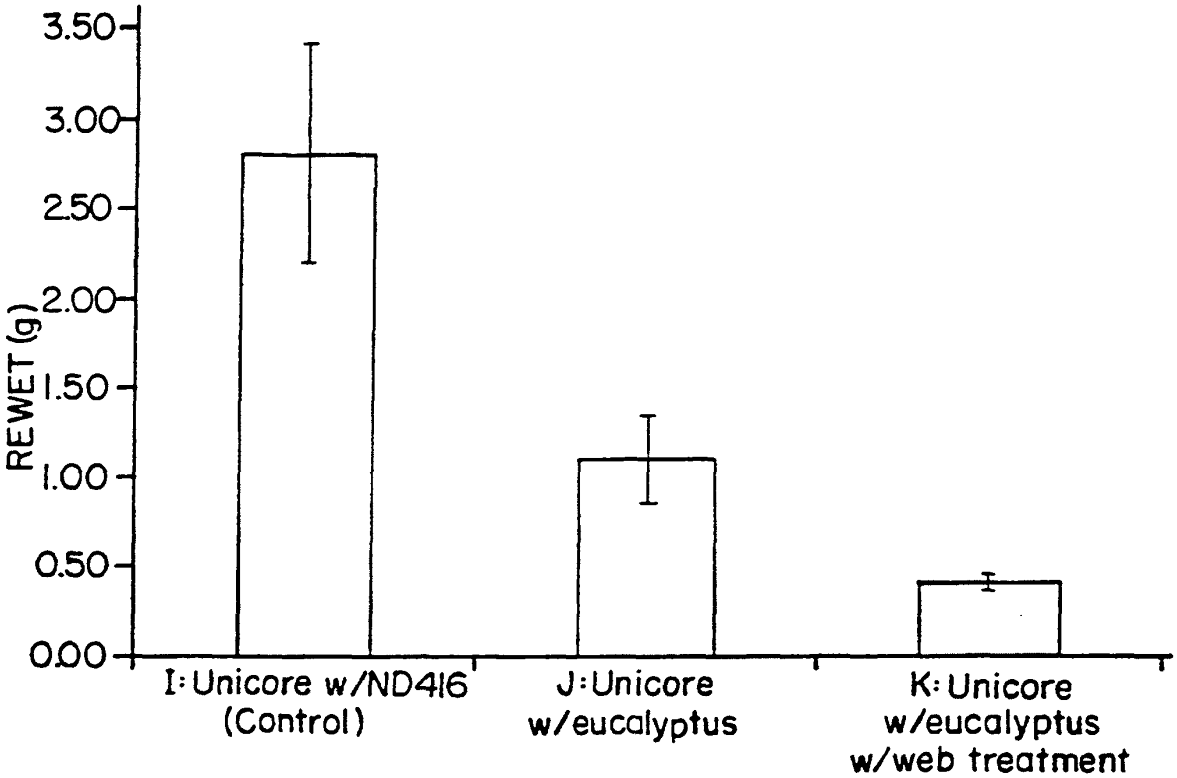


FIG. 9(a)

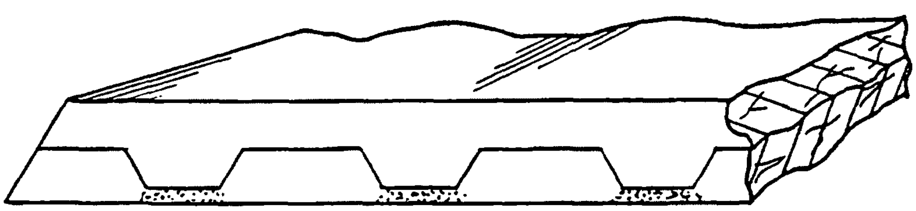

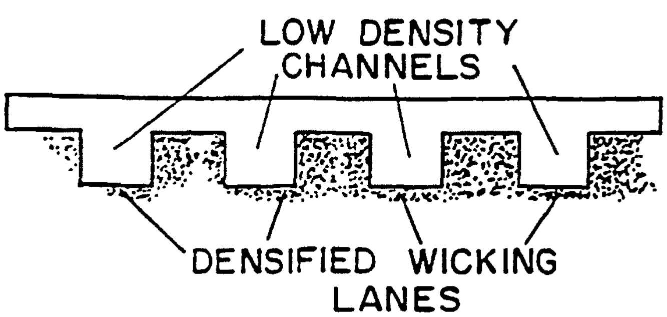


FIG. 9(b)

FIG. 7

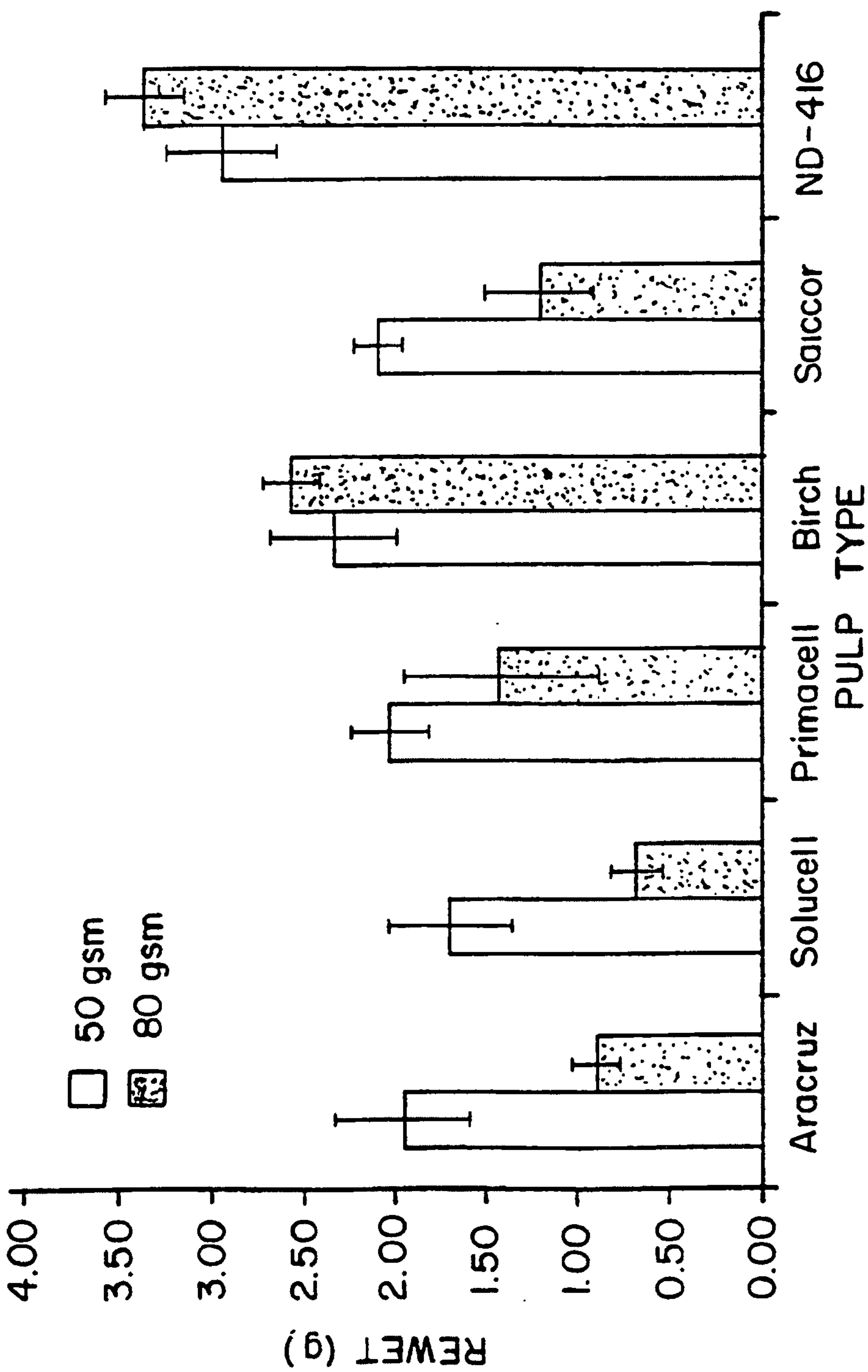
REWET (g)
4.00
3.50
3.00
2.50
2.00
1.50
1.00
0.50
0.00
50 gsm
80 gsm
Aracruz
Solucell
Primacell
Birch
Saiccor
ND-416
PULP TYPE

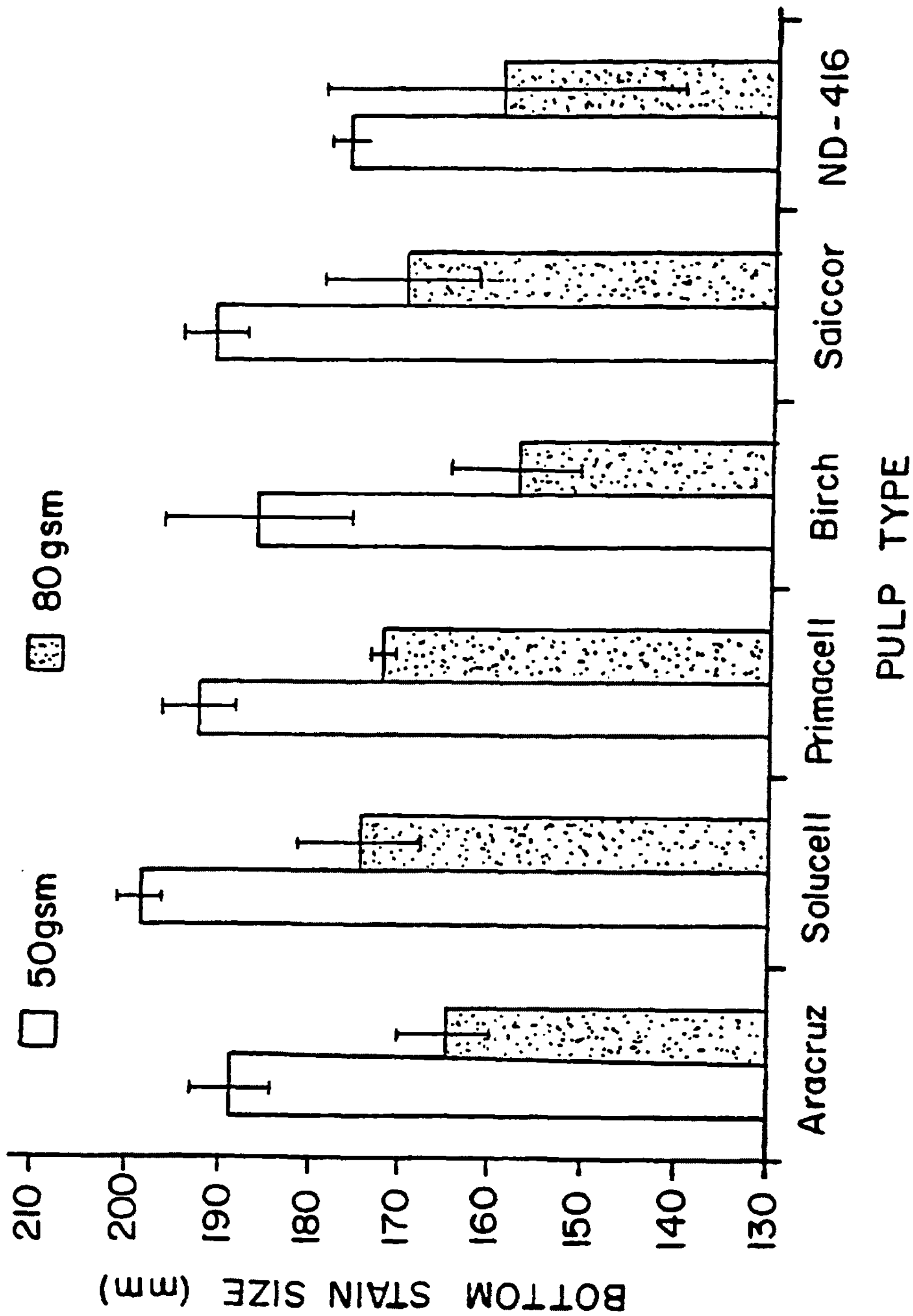
50gsm
80gsm
BOTTOM STAIN SIZE (mm)
210
200
190
180
170
160
150
140
130
Aracruz
Solucell
Primacell
Birch
Saiccor
ND-416
PULP TYPE

FIG. 8